# EUROPEAN PATENT APPLICATION

(11) **EP 4 353 745 A1**
(43) Date of publication of application: **17.04.2024**
(21) Application number: 22819491.6
(22) Date of filing: 06.06.2022
(51) Int. Cl.: C07K 16/28, A61K 39/395, A61K 47/68, A61P 35/00, A61P 37/04

(54) **A GROUP OF B7H3 MONOCLONAL ANTIBODIES AND MEDICAL USE THEREOF**

(30) Priority: 09.06.2021 CN 202110639996
(71) Applicant: INNOLAKE BIOPHARMA (HANGZHOU) CO., LTD, Zhejiang 310018 (CN)
(72) Inventor: QIU, Junzhuan, Hangzhou, Zhejiang 310018 (CN); WANG, Zhensheng, Hangzhou, Zhejiang 310018 (CN); SUN, Kai, Hangzhou, Zhejiang 310018 (CN); CHEN, Junyong, Hangzhou, Zhejiang 310018 (CN); SUN, Jian, Hangzhou, Zhejiang 310018 (CN); LI, Zhongliang, Hangzhou, Zhejiang 310018 (CN); XIA, Mingde, Hangzhou, Zhejiang 310018 (CN); CHEN, Rulei, Hangzhou, Zhejiang 310018 (CN)
(74) Representative: Gassner, Birgitta
(86) International application number: PCT/CN2022/097193
(87) International publication number: WO 2022/257893

(57) **Abstract**

Provided are a group of anti-B7H3 monoclonal antibodies and humanized antibodies thereof. The antibodies obtained by means of hybridoma technology have B7H3 affinity, cell endocytosis effect, and antibody-dependent cytotoxicity, or can effectively block an immunosuppressive effect caused by B7H3, and can be used for preparing related drugs for inhibiting cancer cells, regulating the effect and level of B7H3, and enhancing the immunity of an organism.

## Description

### CROSS-REFERENCE TO RELATED APPLICATIONS

This application claims priority to Chinese Patent Application No. 202110639996.0, filed with the China National Intellectual Property Administration on June 9, 2021 and entitled "A GROUP OF B7H3 MONOCLONAL ANTIBODIES AND MEDICAL USE THEREOF", which is incorporated herein by reference in its entirety.

### TECHNICAL FIELD

The present disclosure belongs to the field of anti-tumor antibody treatment and molecular immunology treatment, and relates to anti-B7H3 antibodies and use thereof. Specifically, the present disclosure relates to various anti-B7H3 monoclonal antibodies.

### BACKGROUND

B7H3, also known as CD276, is a type I transmembrane protein (Chapoval A. I., et al., (2001), Nat. Immunol. 2:269). In mice, B7H3 is encoded by chromosome 9, while in humans, it is encoded by chromosome 15. B7H3 shares 20%-27% amino acid homology with other B7 family ligands (Sun M., et al., (2002), J. Immunol. 168:6294; Loos M., (2010), Clin. Dev. Immunol. 2010:683875). In terms of protein composition and structure, murine B7H3 has only two IgB7H3 types (consisting of a pair of extracellular IgV and IgC), and human B7H3 has four IgB7H3 types, which are almost identical tandem repeats of IgV-IgC (Sun M., et al., (2002), J. Immunol. 168:6294; Steinberger P., et al., (2004), J. Immunol. 172:2352). Although B7H3 mRNA is present in some normal tissues such as liver, small intestine, pancreas, testis, heart and colon, B7H3 protein is rarer in normal tissues (Greenwald R. J., et al., (2005), Annu. Rev. Immunol. 23:515-48). This difference between B7H3 mRNA and protein may reflect existence of close post-transcriptional regulatory mechanisms (Hofmeyer K. A., et al., (2008), Proc. Natl. Acad. Sci. 105:10277; Calabro L., et al., (2011), J. Cell Physiol. 226:2595). Generally, B7H3 is only present in some non-immune fibroblasts, endothelial cells, and osteoblasts, as well as some immune cells such as B cells, T cells, monocytes, dendritic cells, or NK cells. Expression of B7H3 can be induced by CSF or lipopolysaccharide stimulation in granulocyte-macrophage cells (Suh W. K., et al., (2003), Nat. Immunol. 4:899; Chapoval A. I., et al., (2001), Nat. Immunol. 2:269; Greenwald R. J. et al., (2005), Annu. Rev. Immunol. 23:515).

B7H3 belongs to important immune checkpoint proteins. Preliminary studies have shown that B7H3 has a stimulation effect on T cells. It can promote proliferation of CD4 + and CD8 + T cells to some extent, enhance the cytotoxicity of T lymphocytes, and stimulate production of interferon-γ (IFN-γ) (Chapoval A. I., et al., (2001), Nat. Immunol. 2:269). And deficiency of B7H3 can alleviate chronic rejection of cardiac allografts (Wang L. et al., (2005), Eur. J. Immunol. 35:428). However, more studies have shown that B7H3 also has significant immunosuppressive effects. For example, B7H3 can significantly inhibit activation effects of CD3 antibodies or allogeneic DC cells on T cells, and B7H3-blocking antibodies can effectively reverse the inhibitory effect (Prasad D. V. R., et al., (2004), J. Immunol. 173 :2500). Structural information of B7H3 protein reveals that FG loop in B7H3 IgV domain may have a critical effect on inhibition of T cells (Vigdorovich V., et al., (2013), Structure 21 :707). In addition to T cells, B7H3 may also have an inhibitory effect on natural killer cells (NK) (Castriconi R., et al., (2004), Proc. Natl. Acad. Sci. 101: 12640). A possible mechanism is that regulatory T cells (Tregs) induce the expression of B7H3 in dendritic cells (DC), thereby inhibiting a stimulatory effect of DC cells on T lymphocytes (Mahnke K., et al., (2007), Eur. J. Immunol. 37:2117). Mouse models lacking B7H3 exhibit more severe airway inflammations and earlier occurrence of some autoimmune diseases such as experimental autoimmune strain encephalomyelitis (Suh W. K., et al., (2003), Nat. Immunol. 4: 899).

B7H3 is highly expressed in many malignant tumors such as melanoma (Wang J., et al., (2013), J. Invest. Dermatol. 133:2050), leukemia (Hu Y, et al., (2015), Hematology 20:187; Sun J., et al., (2014), OncoTargets Ther. 7:1979), prostate cancer (Zang X., et al., (2007), Proc. Natl. Acad. Sci. 104:19458), ovarian cancer (Zang X., et al., (2010), Mod. Pathol. 23:1104) and pancreatic cancer (Chen Y, et al., (2014), Onco. Targets Ther. 7:1465-72). Although B7H3 mRNA exists in both normal and tumor tissues, B7H3 protein levels are higher in only tumor cells. This difference may be related to a regulatory mechanism of miRNA-29. For example, studies have shown that there is an inverse relationship between miRNA-29 and B7H3 protein level in normal or tumor tissues and cancer cell lines. (Xu H., et al., (2009), Cancer Res. 69:6275). In tumor patients, abnormal expression of B7H3 is closely associated with poor prognosis, increased tumor grade and metastasis, treatment resistance, and decreased overall survival rate of tumor patients (Picarda E., et al., (2016), Clin. Cancer Res. 22:3425).

The inhibitory effect of B7H3 on T cells, NK cells and DC cells significantly promotes immune evasion of tumor cells. In addition, B7H3 plays an important role in proliferation, migration, invasion, angiogenesis, epithelial to mesenchymal transition (EMT), cancer stemness and Warburg effect of tumor cells and tumor cell drug resistance. An increase in B7H3 can promote expression of Bcl-2 and Bcl-xl, to activate a JAK2-STAT3 signaling pathway, thereby enhancing anti-apoptotic activity of tumor cells (Zhang T., et al., (2015), World J. Gastroenterol. 21:1804). In addition, activation of AKT, ERK and JAK2/STAT3 pathways can induce expression of tumor cell metastasis-related protein factors, including MMP2, MMP9, CXCR4, and the like, thereby enhancing migration and aggressiveness of tumor cells (Tekle C., et al., (2013), Int. J Cancer 130:2282; Li Y, et al., (2017), Oncotarget 8:71725; Wang L., et al., (2013), PLoS One 8: e70689; Liu F., et al., (2015), Mol. Med. Rep. 12:5455). Multiple studies have shown that B7H3 in tumor endothelial cells can also induce activation of NF-κB through a TLR4-dependent mechanism, which significantly increases expression levels of VEGF and IL-8 (Tekle C., et al., (2012), Int. J. Cancer 130:2282), thereby promoting aggressiveness and angiogenesis of tumors (Ferrara N., et al., (2002), Nat. Rev. Cancer 2:795). In addition, B7H3 can also regulate activity of tumor cells EMT and cancer stem cells by reducing expression of E-cadherin and increasing expression of N-cadherin, vimentin, CD133 and CD44 (Jiang B., et al., (2016), Oncotarget 731755). Because B7H3 can activate STAT3 signaling to increase expression of hexokinase 2, B7H3 also has an effect of promoting aerobic glycolysis in tumor cells (Shi T., et al., (2019), Cell Death Dis. 10:308 ). In addition, B7H3 inhibits activity of transcription factor NRF2, which increases levels of reactive oxygen species (ROS) and HIF1a, thereby further enhancing the aerobic glycolysis effect of tumor cells and promoting growth of tumor cells (Lim S., et al., (2016), Cancer Res. 76:2231). An increasing number of studies have shown that inhibiting or lowering expression of B7H3 can increase response of tumor cells to some drugs, for example, drugs for inhibiting DNA replication (including alkylation drugs) and drugs for inhibiting PI3K/Akt/mTOR and Ras/Raf/MEK signaling proteins (Flem-Karlsen K., et al., (2017), Pigment Cell Melanoma Res. 30:467; Kasten B. B., et al., (2017), Nucl. Med. Biol. 47:23; Liu H., et al., (2011), Mol. Cancer Ther. 10:960).

Transplanting tumor cells into mice lacking B7H3 or treating tumor mice with B7H3 antibodies significantly inhibits tumor growth (Cai D., et al., (2020), Cell. Mol. Immunol. 17:227; Lee Y. H., et al., (2017), Cell Res. 27:1034), indicating that blocking B7H3 signaling can be used for tumor treatment. In addition, using B7H3 antibodies in combination with anti-PD-1 antibodies can cause dual blockade of B7H3 and PD-1, thereby exerting a synergistic anti-tumor effect (Lee Y. H., et al., (2017), Cell Res. 27:1034 ). Due to a significant difference between B7H3 expression levels of normal tissues and tumor tissues, tumor cells can be effectively killed through the ADCC effect of B7H3 antibodies or toxin conjugation without causing excessive impact on normal tissues (Koenig S., et al., (2014), Medicographia 36:285). In summary, based on current research, B7H3 is a promising target for cancer treatment.

### SUMMARY

In the present disclosure, a group of anti-B7H3 monoclonal antibodies were obtained through hybridoma technology. These antibodies have high B7H3 affinity, significant cellular internalization (allowing selective tumor killing or inhibition through toxin conjugation), significant antibody-dependent cytotoxicity (ADCC), or effective blocking of the immunosuppressive action of B7H3. In the present disclosure, six candidate antibodies were successfully humanized. These antibodies have extensive potential in developing drugs to inhibit cancer cells, regulate the action and levels of B7H3, and enhance immune response, especially in cancer treatment.

A murine or humanized B7H3 antibody or a functional fragment thereof provided in the present disclosure includes a heavy chain sequence and a light chain sequence. For anti-human B7H3 murine antibodies, the amino acid sequence information of the heavy chain variable regions and the light chain variable regions is as follows: The amino acid sequences of 7F5's heavy chain variable region and light chain variable region are SEQ ID NO: 1 and 2, respectively; the amino acid sequences of 9C8's heavy chain variable region and light chain variable region are SEQ ID NO: 3 and 4, respectively; the amino acid sequences of 5B6's heavy chain variable region and light chain variable region are SEQ ID NO: 5 and 6, respectively; the amino acid sequences of 7C9's heavy chain variable region and light chain variable region are SEQ ID NO: 7 and 8, respectively; the amino acid sequences of 2A9's heavy chain variable region and light chain variable region are SEQ ID NO: 9 and 10, respectively; the amino acid sequences of 4F11's heavy chain variable region and light chain variable region are SEQ ID NO: 11 and 12, respectively; the amino acid sequences of 15A2's heavy chain variable region and light chain variable region are SEQ ID NO: 13 and 14, respectively; the amino acid sequences of 7B7's heavy chain variable region and light chain variable region are SEQ ID NO: 15 and 16, respectively; the amino acid sequences of 7E6's heavy chain variable region and light chain variable region are SEQ ID NO: 17 and 18, respectively; the amino acid sequences of 2E10's heavy chain variable region and light chain variable region are SEQ ID NO: 19 and 20, respectively; the amino acid sequences of 2F12's heavy chain variable region and light chain variable region are SEQ ID NO: 21 and 22, respectively; the amino acid sequences of 2F7's heavy chain variable region and light chain variable region are SEQ ID NO: 23 and 24, respectively; the amino acid sequences of 13A2's heavy chain variable region and light chain variable region are SEQ ID NO: 25 and 26, respectively; and the amino acid sequences of 14B3's heavy chain variable region and light chain variable region are SEQ ID NO: 27 and 28, respectively.

The amino acid sequence information of CDR1, CDR2, and CDR3 of the foregoing heavy chains and light chains of the antibodies is as follows: The amino acid sequences of 7F5's heavy chain CDR1, CDR2, and CDR3 are SEQ ID NO: 29, 30, and 31, respectively, and the amino acid sequences of 7F5's light chain CDR1, CDR2, and CDR3 are SEQ ID NO: 32, 33, and 34, respectively; the amino acid sequences of 9C8's heavy chain CDR1, CDR2, and CDR3 are SEQ ID NO: 35, 36, and 37, respectively, and the amino acid sequences of 9C8's light chain CDR1, CDR2, and CDR3 are SEQ ID NO: 38, 39, and 40, respectively; the amino acid sequences of 5B6's heavy chain CDR1, CDR2, and CDR3 are SEQ ID NO: 41, 42, and 43, respectively, and the amino acid sequences of 5B6's light chain CDR1, CDR2, and CDR3 are SEQ ID NO: 44, 45, and 46, respectively; the amino acid sequences of 7C9's heavy chain CDR1, CDR2, and CDR3 are SEQ ID NO: 47, 48, and 49, respectively, and the amino acid sequences of 7C9's light chain CDR1, CDR2, and CDR3 are SEQ ID NO: 50, 51, and 52, respectively; the amino acid sequences of 2A9's heavy chain CDR1, CDR2, and CDR3 are SEQ ID NO: 53, 54, and 55, respectively, and the amino acid sequences of 2A9's light chain CDR1, CDR2, and CDR3 are SEQ ID NO: 56, 57, and 58, respectively; the amino acid sequences of 4F11's heavy chain CDR1, CDR2, and CDR3 are SEQ ID NO: 59, 60, and 61, respectively, and the amino acid sequences of 4F11's light chain CDR1, CDR2, and CDR3 are SEQ ID NO: 62, 63, and 64, respectively; the amino acid sequences of 15A2's heavy chain CDR1, CDR2, and CDR3 are SEQ ID NO: 65, 66, and 67, respectively, and the amino acid sequences of 15A2's light chain CDR1, CDR2, and CDR3 are SEQ ID NO: 68, 69, and 70, respectively; the amino acid sequences of 7B7's heavy chain CDR1, CDR2, and CDR3 are SEQ ID NO: 71, 72, and 73, respectively, and the amino acid sequences of 7B7's light chain CDR1, CDR2, and CDR3 are SEQ ID NO: 74, 75, and 76, respectively; the amino acid sequences of 7E6's heavy chain CDR1, CDR2, and CDR3 are SEQ ID NO: 77, 78, and 79, respectively, and the amino acid sequences of 7E6's light chain CDR1, CDR2, and CDR3 are SEQ ID NO: 80, 81, and 82, respectively; the amino acid sequences of 2E10's heavy chain CDR1, CDR2, and CDR3 are SEQ ID NO: 83, 84, and 85, respectively, and the amino acid sequences of 2E10's light chain CDR1, CDR2, and CDR3 are SEQ ID NO: 86, 87, and 88, respectively; the amino acid sequences of 2F12's heavy chain CDR1, CDR2, and CDR3 are SEQ ID NO: 89, 90, and 91, respectively, and the amino acid sequences of 2F12's light chain CDR1, CDR2, and CDR3 are SEQ ID NO: 92, 93, and 94, respectively; the amino acid sequences of 2F7's heavy chain CDR1, CDR2, and CDR3 are SEQ ID NO: 95, 96, and 97, respectively, and the amino acid sequences of 2F7's light chain CDR1, CDR2, and CDR3 are SEQ ID NO: 98, 99, and 100, respectively; the amino acid sequences of 13A2's heavy chain CDR1, CDR2, and CDR3 are SEQ ID NO: 101, 102, and 103, respectively, and the amino acid sequences of 13A2's light chain CDR1, CDR2, and CDR3 are SEQ ID NO: 104, 105, and 106, respectively; and the amino acid sequences of 14B3's heavy chain CDR1, CDR2, and CDR3 are SEQ ID NO: 107, 108, and 109, respectively, and the amino acid sequences of 14B3's light chain CDR1, CDR2, and CDR3 are SEQ ID NO: 110, 111, and 112, respectively.

Further, the anti-human B7H3 antibody or fragment is modified to become a humanized antibody.

An amino acid sequence of a heavy chain variable region of anti-human B7H3 humanized antibody 2A9 is SEQ ID NO: 113, and an amino acid sequence of a light chain variable region of anti-human B7H3 humanized antibody 2A9 is SEQ ID NO: 114; an amino acid sequence of a heavy chain variable region of humanized antibody 4F11 is SEQ ID NO: 115, and an amino acid sequence of a light chain variable region of humanized antibody 4F11 is SEQ ID NO: 116; an amino acid sequence of a heavy chain variable region of humanized antibody 9C8 is SEQ ID NO: 117, and an amino acid sequence of a light chain variable region of humanized antibody 9C8 is SEQ ID NO: 118; an amino acid sequence of a heavy chain variable region of humanized antibody 15A2 is SEQ ID NO: 119, and an amino acid sequence of a light chain variable region of humanized antibody 15A2 is SEQ ID NO: 120; an amino acid sequence of a heavy chain variable region of humanized antibody 5B6 is SEQ ID NO: 121, and an amino acid sequence of a light chain variable region of humanized antibody 5B6 is SEQ ID NO: 122; and an amino acid sequence of a heavy chain variable region of humanized antibody 7B7 is SEQ ID NO: 123, and an amino acid sequence of a light chain variable region of humanized antibody 7B7 is SEQ ID NO: 124.

An expression vector includes a nucleic acid molecule of the antibody.

A pharmaceutical composition includes the antibody or a functional fragment thereof, and a pharmaceutical carrier.

Use of the antibody or the functional fragment, nucleic acid molecule, expression vector, host cell, and pharmaceutical composition thereof in preparing B7H3 immunomodulatory medications is provided.

In the present disclosure, recombinant B7H3 receptor proteins produced in a mammalian cell expression system are used as antigens to immunize mice, followed by fusing the mice's splenocytes with myeloma cells to obtain hybridoma cells. Multiple monoclonal hybridoma cell lines are obtained through extensive cloning and screening of these hybridoma cells. These hybridoma cell lines can secrete monoclonal antibodies (FIG. 1 and FIG. 2) that specifically bind to B7H3 receptors. Some of these monoclonal antibodies have a significant effect of being endocytosed by cells (FIG. 3 and FIG. 4), some have an effect of promoting human immune cells to secrete cytokines such as IFN-γ (FIG. 5), and some have a significant effect of mediating ADCC (FIG. 6, FIG. 7 and FIG. 8). Some monoclonal antibodies are potential candidates for future development of ADC, ADCC, and B7H3 antagonists. The invention further involves cloning the genes encoding antibody light and heavy chain variable regions through RT-PCR (Reverse Transcription-Polymerase Chain Reaction), and constructing humanized antibodies using the complementarity-determining regions graft (CDR-graft) method. *In vitro* functional experiments show that the humanized B7H3 antibodies can specifically bind to B7H3 receptor proteins (FIG. 9 and FIG. 10) while maintaining significant cellular internalization (FIG. 11), have a significant cytotoxic effect on Calu-6 tumor cells post-MMAE toxin conjugation (FIG. 12), an ADCC mediation effect (FIG. 13), a mediation effect on ADCC cytotoxic effect of NK92MI-hCD16 cells on Jurkat-B7H3 and PC9 cells (FIG. 14), and an effect of promoting human immune cells to produce and secret cytokines such as IFN-γ (FIG. 15). The positive control antibodies used in the present disclosure are derived from patent applications US8802091 and WO2017180813A1.

### BRIEF DESCRIPTION OF THE DRAWINGS

In order to illustrate the technical solutions in the specific embodiments of the present disclosure or in the prior art more clearly, the following briefly describes the accompanying drawings used for describing the specific embodiments or the prior art. Apparently, the accompanying drawings in the following description show only some embodiments of the present disclosure, and those of ordinary skills in the art may derive other drawings from these accompanying drawings without creative efforts.

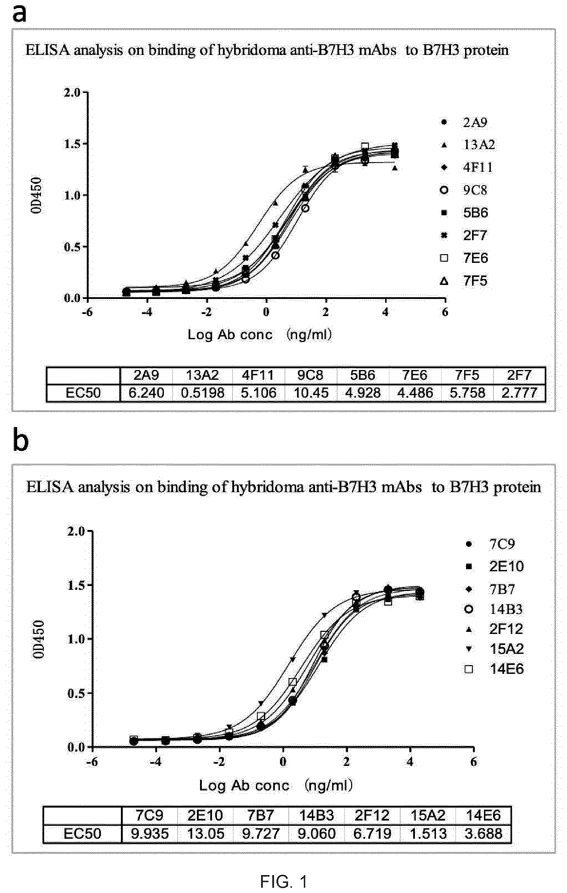

FIG. 1(a-b): Determination of the binding properties of B7H3 hybridoma monoclonal antibodies with B7H3-hFc protein using ELISA method;

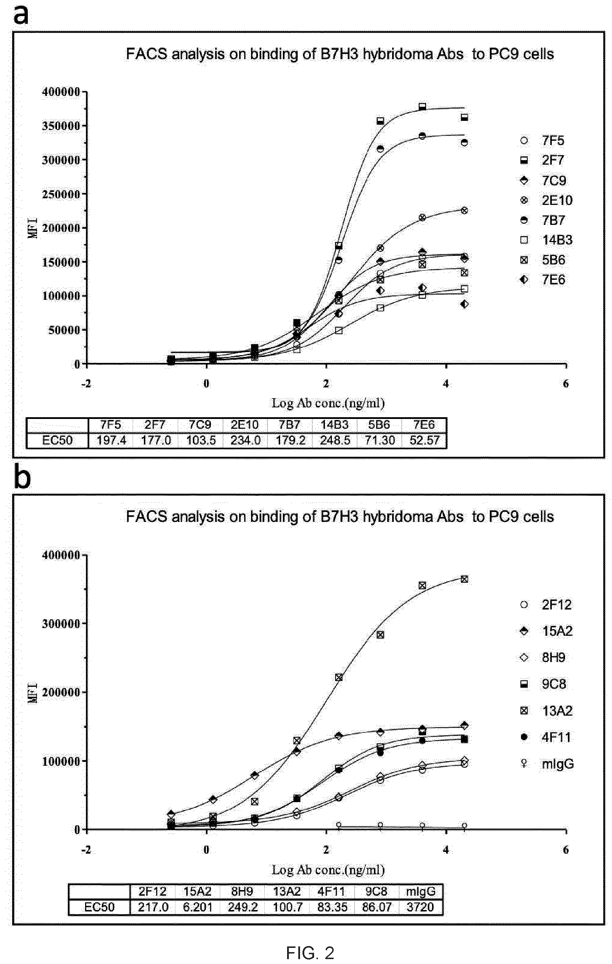

FIG. 2(a-b): Determination of the binding properties of B7H3 hybridoma monoclonal antibodies with PC9 cells using FACS method;

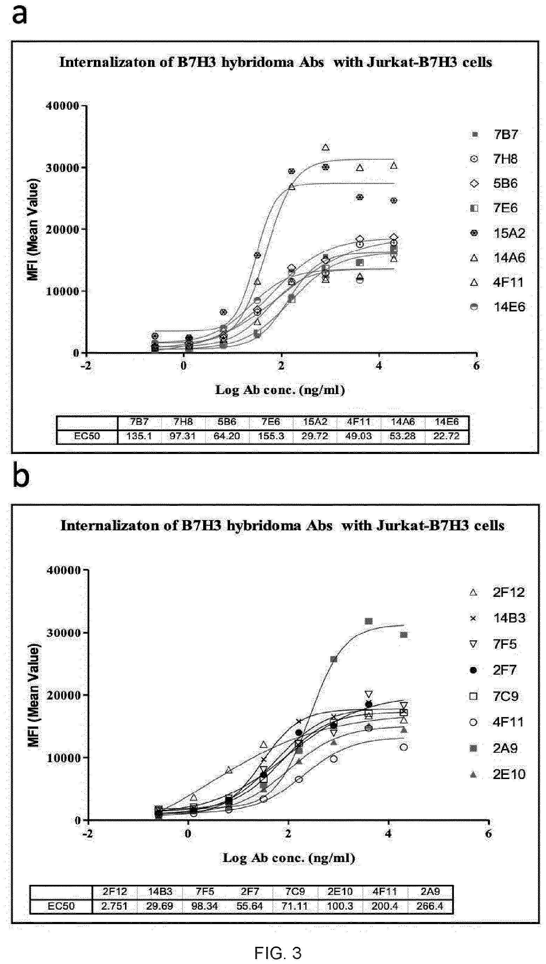

FIG. 3(a-b): Determination of the cellular internalization activity of B7H3 hybridoma antibodies by Jurkat-B7H3 cells through FACS;

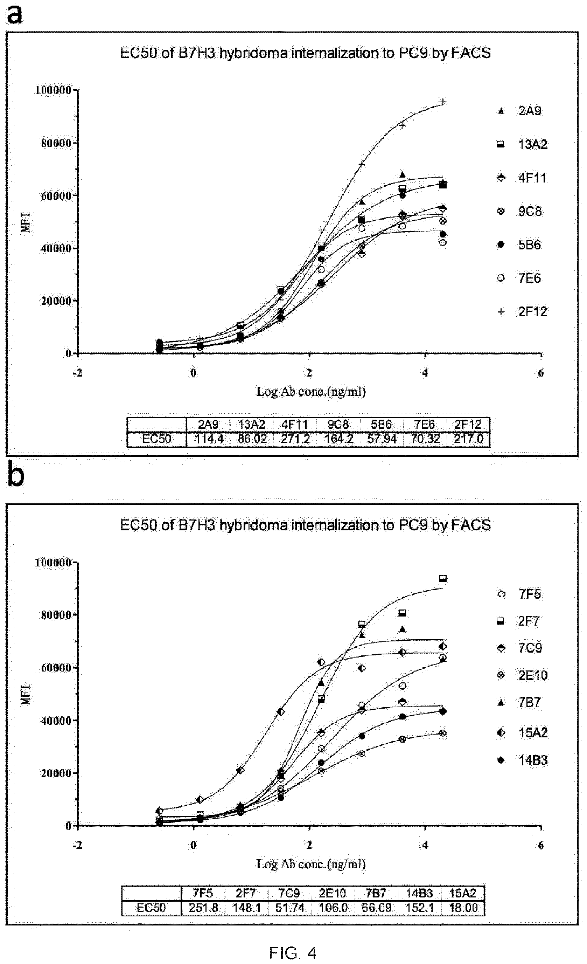

FIG. 4(a-b): Determination of the cellular internalization activity of B7H3 hybridoma antibodies by PC9 cells through FACS;

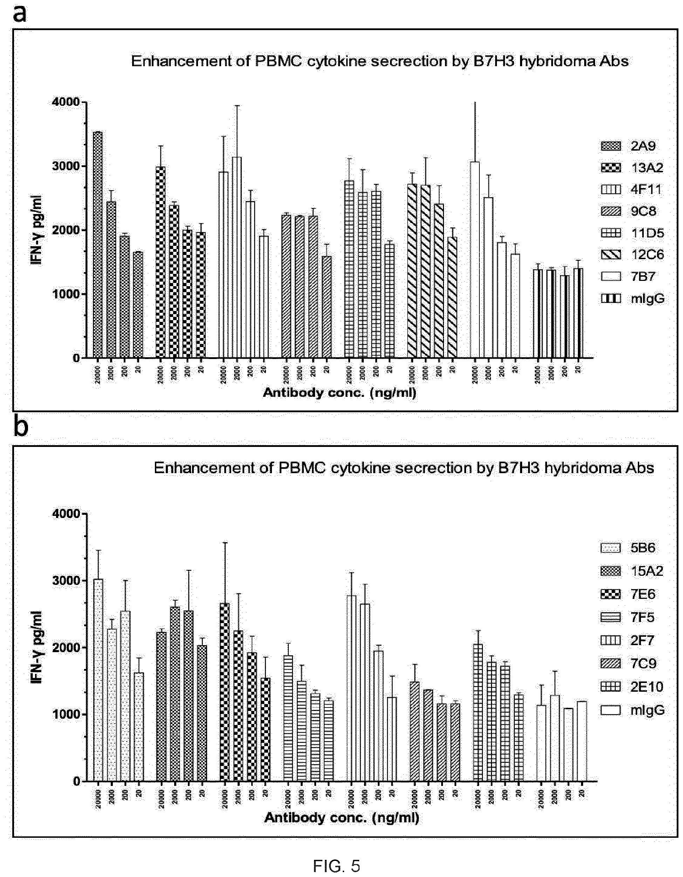

FIG. 5(a-b): Promoting effect of B7H3 hybridoma antibodies on IFN-γ secretion in human PBMC cells;

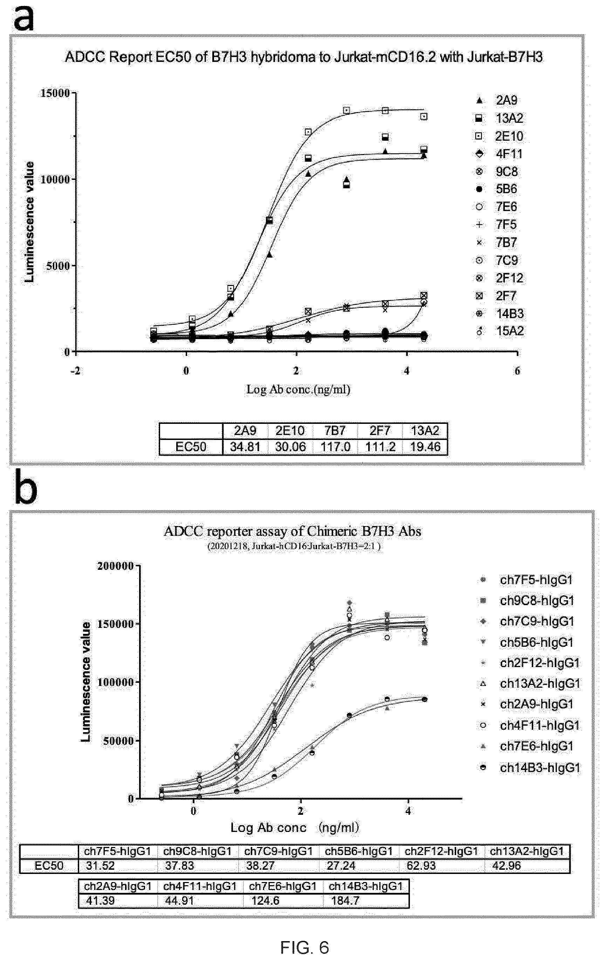

FIG. 6(a-b): Testing of the ADCC effect on Jurkat-B7H3 cells mediated by B7H3 hybridoma or chimeric antibodies using the reporter gene method;

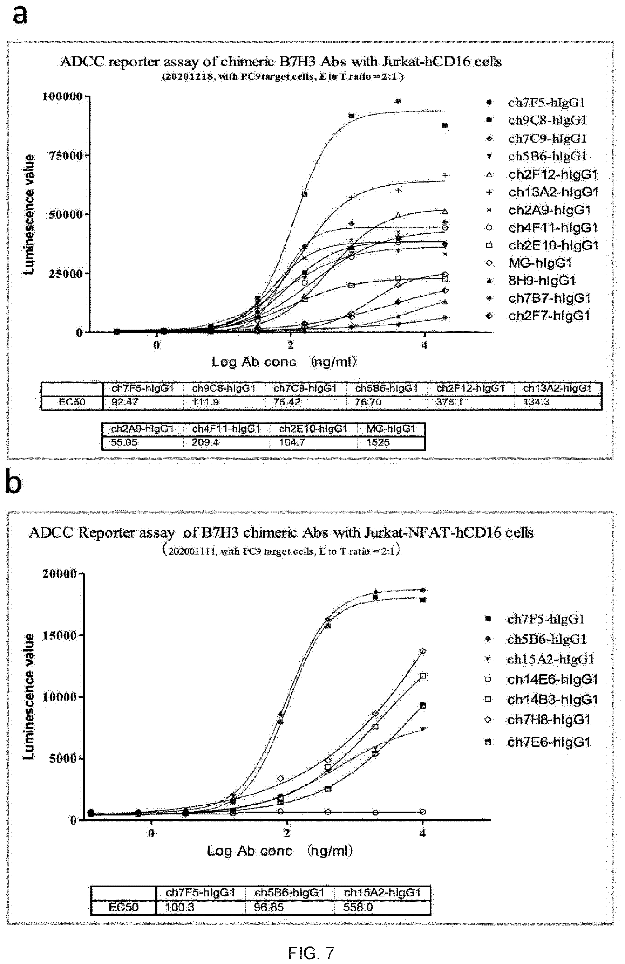

FIG. 7(a-b): Testing of the ADCC effect on PC9 tumor cells mediated by some B7H3 chimeric antibodies using the reporter gene method;

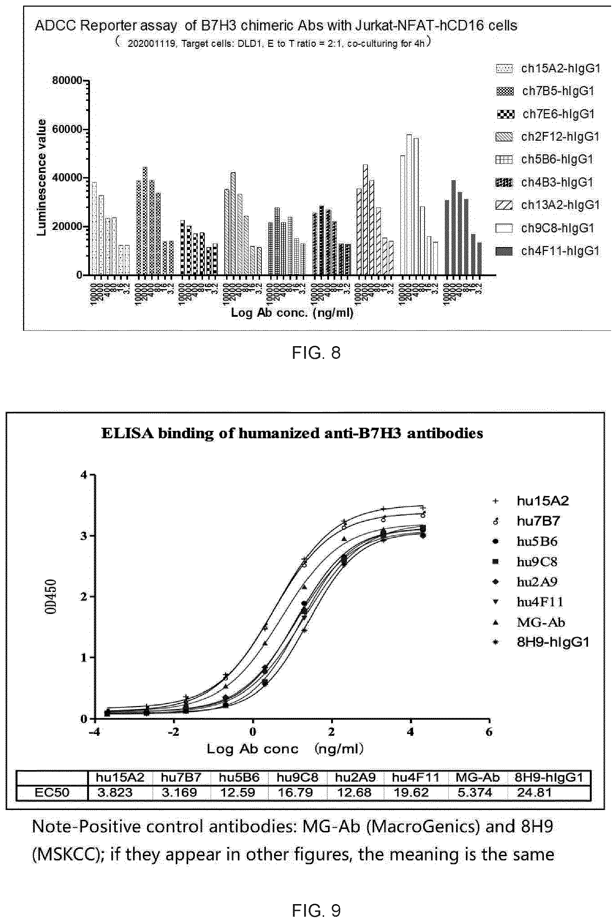

FIG. 8: Testing of the ADCC effect on DLD1 tumor cells mediated by some B7H3 chimeric antibodies using the reporter gene method;
FIG. 9: Determination of the binding properties of humanized B7H3 monoclonal antibodies with B7H3-hFc protein using ELISA method;

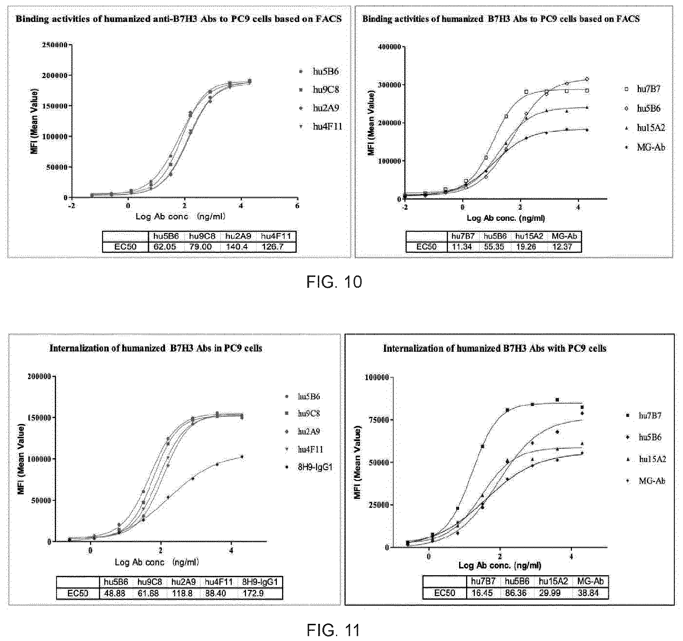

FIG. 10: Determination of the binding properties of humanized B7H3 monoclonal antibodies with B7H3 protein in PC9 cells using FACS method;
FIG. 11: Determination of the cellular internalization effect of humanized B7H3 monoclonal antibodies by PC9 cells through FACS;

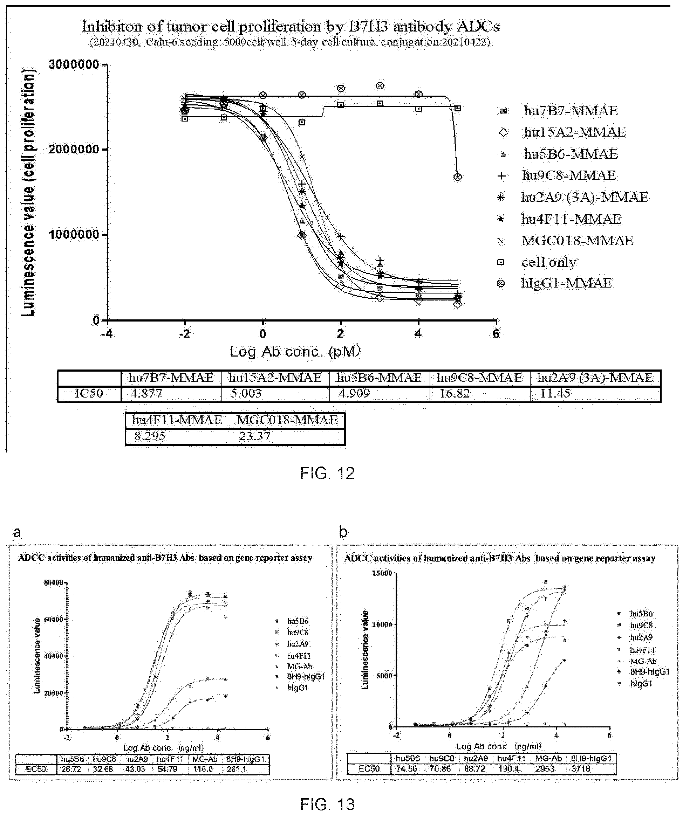

FIG. 12: Cytotoxic effect of humanized B7H3 antibody ADC on Calu-6 tumor cells;
FIG. 13(a-b): Testing of the ADCC effect of humanized B7H3 antibodies on Jurkat-B7H3 and PC9 cells using the reporter gene method;

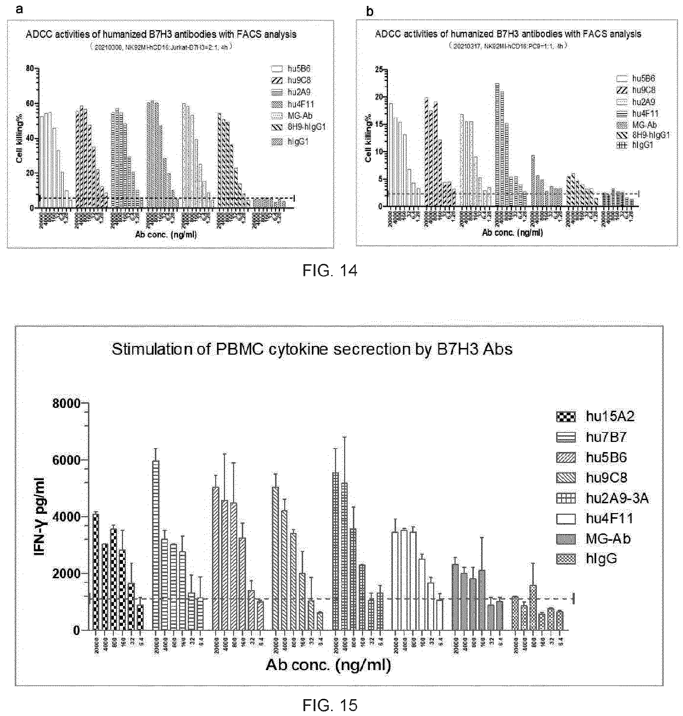

FIG. 14(a-b): ADCC cytotoxic effect of NK92MI-hCD16 cells on Jurkat-B7H3 and PC9 cells mediated by humanized B7H3 antibodies; and
FIG. 15: Promoting effect of humanized B7H3 monoclonal antibodies on IFN-γ production and secretion by human PBMC cells.

### DETAILED DESCRIPTION

As used herein, the term "antibody" is used in its broadest sense and includes immunoglobulin or other molecules containing one or more antigen-binding domains that specifically bind to an antigen, namely, a protein or peptide that exhibit binding specificity for a particular antigen. Specific examples of antibodies may include intact antibodies (for example, classic four-chain antibody molecules), single-chain antibodies, single-domain antibodies, bispecific antibodies, multispecific antibodies, and the like. A classic antibody molecule is usually a tetramer composed of two identical heavy chains and two identical light chains connected to each other through disulfide bonds. Based on a conservative difference in amino acid sequences, heavy and light chains are divided into variable regions (V) located at amino terminals and constant regions (C) located at carboxyl terminals. The variable region is used to recognize and bind the antigen, and the constant region (for example, Fc fragment) is used to initiate a downstream effect, for example, cell-mediated antibody-dependent cytotoxicity (ADCC) effect. Within the variable regions of the heavy chain and light chain, three local regions have a higher degree of variation in amino acid composition and sequences, are key binding positions for antibodies and antigens, and therefore, are also referred to as complementarity-determining regions (CDRs). The amino acid sequences of the CDRs can be readily determined by using numbering schemes recognized in the field, for example, Kabat, Chothia, IMGT, AbM or Contact.

An "antigen-binding fragment" of an antibody refers to an amino acid fragment in the antibody molecule that participates in specific antigen binding, for example, one of F(ab')₂, Fab and scFv.

EC₅₀ (concentration for 50% of maximal effect) refers to the concentration that causes 50% of the maximal effect. When used to indicate the binding ability of an antibody molecule to a corresponding antigen in an enzyme-linked immunosorbent assay (ELISA), EC₅₀ can refer to concentration of the antibody molecule when half of the maximum detection signal (for example, colorimetric or fluorescence intensity) occurs. The lower the value of EC₅₀, the greater the binding affinity to the antigen.

The present disclosure relates to an anti-B7H3 monoclonal antibody or an antigen-binding fragment thereof, including heavy chains and light chains, where an amino acid sequence of the heavy chain CDR1 is selected from one of SEQ ID NOs: 29, 35, 41, 47, 53, 59, 65, 71, 77, 83, 89, 95, 101 and 107; an amino acid sequence of heavy chain CDR2 is selected from one of SEQ ID NOs: 30, 36, 42, 48, 54, 60, 66, 72, 78, 84, 90, 96, 102 and 108; an amino acid sequence of heavy chain CDR3 is selected from one of SEQ ID NOs: 31, 37, 43, 49, 55, 61, 67, 73, 79, 85, 91, 97, 103 and 109; an amino acid sequence of light chain CDR1 is selected from one of SEQ ID NOs: 32, 38, 44, 50, 56, 62, 68, 74, 80, 86, 92, 98, 104 and 110; an amino acid sequence of light chain CDR2 is selected from one of SEQ ID NOs: 33, 39, 45, 51, 57, 63, 69, 75, 81, 87, 93, 99, 105 and 111; and an amino acid sequence of light chain CDR3 is selected from one of SEQ ID NOs: 34, 40, 46, 52, 58, 64, 70, 76, 82, 88, 94, 100, 106 and 112, wherein the heavy and light chains of the antigen-binding fragment comprise amino acid sequences spanning from CDR1 to CDR3 of respective heavy and light chains of the antibody.

The anti-B7H3 antibody or antigen-binding fragment thereof provided in the present disclosure includes a heavy chain CDR and a light chain CDR, where the heavy chain CDR and the light chain CDR are selected from one or more groups of CDR combinations denoted as a to n:

| Combination name | Heavy chain CDR1 | Heavy chain CDR2 | Heavy chain CDR3 | Light chain CDR1 | Light chain CDR2 | Light chain CDR3 |
|---|---|---|---|---|---|---|
| a | SEQ ID NO: 29 | SEQ ID NO: 30 | SEQ ID NO: 31 | SEQ ID NO: 32 | SEQ ID NO: 33 | SEQ ID NO: 34 |
| b | SEQ ID NO: 35 | SEQ ID NO: 36 | SEQ ID NO: 37 | SEQ ID NO: 38 | SEQ ID NO: 39 | SEQ ID NO: 40 |
| c | SEQ ID NO: 41 | SEQ ID NO: 42 | SEQ ID NO: 43 | SEQ ID NO: 44 | SEQ ID NO: 45 | SEQ ID NO: 46 |
| d | SEQ ID NO: 47 | SEQ ID NO: 48 | SEQ ID NO: 49 | SEQ ID NO: 50 | SEQ ID NO: 51 | SEQ ID NO: 52 |
| e | SEQ ID NO: 53 | SEQ ID NO: 54 | SEQ ID NO: 55 | SEQ ID NO: 56 | SEQ ID NO: 57 | SEQ ID NO: 58 |
| f | SEQ ID NO: 59 | SEQ ID NO: 60 | SEQ ID NO: 61 | SEQ ID NO: 62 | SEQ ID NO: 63 | SEQ ID NO: 64 |
| 9 | SEQ ID NO: 65 | SEQ ID NO: 66 | SEQ ID NO: 67 | SEQ ID NO: 68 | SEQ ID NO: 69 | SEQ ID NO: 70 |
| h | SEQ ID NO: 71 | SEQ ID NO: 72 | SEQ ID NO: 73 | SEQ ID NO: 74 | SEQ ID NO: 75 | SEQ ID NO: 76 |
| i | SEQ ID NO: 77 | SEQ ID NO: 78 | SEQ ID NO: 79 | SEQ ID NO: 80 | SEQ ID NO: 81 | SEQ ID NO: 82 |
| g | SEQ ID NO: 83 | SEQ ID NO: 84 | SEQ ID NO: 85 | SEQ ID NO: 86 | SEQ ID NO: 87 | SEQ ID NO: 88 |
| k | SEQ ID NO: 89 | SEQ ID NO: 90 | SEQ ID NO: 91 | SEQ ID NO: 92 | SEQ ID NO: 93 | SEQ ID NO: 94 |
| l | SEQ ID NO: 95 | SEQ ID NO: 96 | SEQ ID NO: 97 | SEQ ID NO: 98 | SEQ ID NO: 99 | SEQ ID NO: 100 |
| m | SEQ ID NO: 101 | SEQ ID NO: 102 | SEQ ID NO: 103 | SEQ ID NO: 104 | SEQ ID NO: 105 | SEQ ID NO: 106 |
| n | SEQ ID NO: 107 | SEQ ID NO: 108 | SEQ ID NO: 109 | SEQ ID NO: 110 | SEQ ID NO: 111 | SEQ ID NO: 112 |

In some embodiments, an amino acid sequence of a heavy chain variable region is selected from one of SEQ ID NOs: 1, 3, 5, 7, 9, 11, 13, 15, 17, 19, 21, 23, 25 and 27, and an amino acid sequence of a light chain variable region is selected from one of SEQ ID NOs: 2, 4, 6, 8, 10, 12, 14, 16, 18, 20, 22, 24, 26 and 28.

In some embodiments, the anti-B7H3 monoclonal antibody or the antigen-binding fragment thereof includes a heavy chain variable region and a light chain variable region, wherein the heavy chain variable region and the light chain variable region are selected from one or more groups of variable region combinations denoted as A to N:

| Combination name | Heavy chain variable region | Light chain variable region |
|---|---|---|
| A | SEQ ID NO: 1 | SEQ ID NO: 2 |
| B | SEQ ID NO: 3 | SEQ ID NO: 4 |
| C | SEQ ID NO: 5 | SEQ ID NO: 6 |
| D | SEQ ID NO: 7 | SEQ ID NO: 8 |
| E | SEQ ID NO: 9 | SEQ ID NO: 10 |
| F | SEQ ID NO: 11 | SEQ ID NO: 12 |
| G | SEQ ID NO: 13 | SEQ ID NO: 14 |
| H | SEQ ID NO: 15 | SEQ ID NO: 16 |
| I | SEQ ID NO: 17 | SEQ ID NO: 18 |
| J | SEQ ID NO: 19 | SEQ ID NO: 20 |
| K | SEQ ID NO: 21 | SEQ ID NO: 22 |
| L | SEQ ID NO: 23 | SEQ ID NO: 24 |
| M | SEQ ID NO: 25 | SEQ ID NO: 26 |
| N | SEQ ID NO: 27 | SEQ ID NO: 28 |

Based on general understandings of persons skilled in the art, functional variants of the antibody or its antigen-binding fragment are also encompassed within the scope of this disclosure. The term "functional variant" used herein refers to a variant molecule obtained by introducing one or more amino acids for insertion, deletion or substitution based on the parent protein molecule (for example, a native protein molecule), where the variant molecule still retains at least some of the functions of the parent protein molecule (especially a function of interest, for example, an ability to bind to the corresponding antigen).

For the CDR region, the functional variants of the present disclosure include a heavy chain complementarity-determining region and a light chain complementarity-determining region. Compared with any one of the complementation-determining region groups denoted as a to n, the heavy chain complementarity-determining region and the light chain complementarity-determining region each contain mutations of up to 3 amino acids (for example, substitution, deletion or addition of 1, 2 or 3 amino acids or any combination thereof). Preferably, the mutations are conservative mutations.

For the variable region, in some embodiments, the functional variant in the present disclosure includes a heavy chain variable region, and an amino acid sequence of the heavy chain variable region includes a sequence sharing at least 80%, 85%, 90%, 91%, 92%, 93%, 94%, 95%, 96%, 97%, 98% or 99% identity with any one of the heavy chain variable region sequences shown in the groups A to N. In some embodiments, the functional variant in the present disclosure includes a light chain variable region, and an amino acid sequence of the light chain variable region includes a sequence sharing at least 80%, 85%, 90%, 91%, 92%, 93%, 94%, 95%, 96%, 97%, 98% or 99% identity with any one of the light chain variable region sequences shown in the groups A to N. The term "identity" refers to a quantity of a degree of identity between two amino acid or nucleotide sequences (for example, a query sequence and a reference sequence), and is generally expressed as a percentage. Usually, before the percentage of identity between two amino acid or nucleotide sequences is calculated, the two amino acid or nucleotide sequences are first subjected to sequence alignment and a gap (if any) is introduced. At a certain alignment position, if amino acid residues or bases in the two sequences are the same, the two sequences are consistent or matched at the position; or if the amino acid residues or bases in the two sequences are different, the two sequences are considered to be inconsistent or mismatched at the position. In some algorithms, the number of matched positions is divided by the total number of positions in the alignment window to obtain the sequence identity. In other algorithms, the number of gaps and/or gap length is also taken into account. Commonly used sequence alignment algorithms or software include DANMAN, CLUSTALW, MAFFT, BLAST, MUSCLE, among others. For the purposes of the present disclosure, the publicly available alignment software BLAST (available at https://www.ncbi.nlm.nih.gov/) can be used to obtain optimal sequence alignment through default settings and calculate the sequence identity between two amino acid or nucleotide sequences.

The functional variant retains the ability to specifically bind B7H3. Persons skilled in the art can determine a suitable variant of the antigen-binding molecule illustrated herein by using well-known technologies. In certain embodiments, persons skilled in the art can identify regions in the antibody or antigen-binding fragment thereof that are unimportant for the activity of specifically binding B7H3 and change suitable regions without destroying the activity.

In some embodiments, the B7H3 monoclonal antibody or antigen-binding fragment thereof is murine or humanized.

In some embodiments, the heavy chain variable region and light chain variable region are humanized.

In some embodiments, the heavy and light chains are humanized.

The term "humanized antibody", also known as CDR-grafted antibody, refers to an antibody produced by transplanting the first animal-derived CDR sequence into human antibody variable region frameworks, that is, different types of human germline antibody framework sequences. Humanized antibodies can overcome the heterologous reactions induced by chimeric antibodies due to their significant murine protein content. Such framework sequences can be obtained from public DNA databases or published references containing germline antibody gene sequences. For example, germline DNA sequences of human heavy and light chain variable region genes are available in "VBase" human germline sequence database (www.mrccpe.com.ac.uk/vbase) and found in Sequences of Proteins of Immunological Interest (Kabat, E.A. et al., 1991, 5th ed.) To avoid both a decrease in immunogenicity and a resulting decrease in activity, a minimum of reverse mutation or back mutation can be performed on the human antibody variable region framework sequence to maintain activity. The humanized antibody in the present disclosure also includes a mature humanized antibody obtained after phage shows affinity to the CDR. In a preferred embodiment of the present disclosure, the first animal origin is of murine origin.

In some embodiments, the heavy chain and the light chain are humanized; an amino acid sequence of a humanized heavy chain variable region is selected from one of SEQ ID NOs: 113, 115, 117, 119, 121, and 123; and the amino acid sequence of the humanized light chain variable region is selected from one of SEQ ID NOs: 114, 116, 118, 120, 122, and 124.

In some embodiments, the humanized heavy chain variable region and light chain variable region are selected from one or more groups of variable region combinations denoted as 1 to 6:

| Combination name | Heavy chain variable region | Light chain variable region |
|---|---|---|
| 1 | SEQ ID NO: 113 | SEQ ID NO: 114 |
| 2 | SEQ ID NO: 115 | SEQ ID NO: 116 |
| 3 | SEQ ID NO: 117 | SEQ ID NO: 118 |
| 4 | SEQ ID NO: 119 | SEQ ID NO: 120 |
| 5 | SEQ ID NO: 121 | SEQ ID NO: 122 |
| 6 | SEQ ID NO: 123 | SEQ ID NO: 124 |

In some embodiments, the heavy chain and the light chain are humanized; an amino acid sequence of a humanized heavy chain variable region is an amino acid sequence with SEQ ID NO: 113; and the amino acid sequence of the humanized light chain variable region is an amino acid sequence with SEQ ID NO: 114.

In some embodiments, the anti-B7H3 monoclonal antibody or the antigen-binding fragment thereof includes a constant region of human IgG1.

The present disclosure further relates to use of the anti-B7H3 monoclonal antibody or the antigen-binding fragment thereof in preparation of the following medication: a medication for regulating B7H3 activity or B7H3 level, a medication that has significant cellular internalization (allowing selective tumor killing or inhibition through toxin conjugation), significant antibody-dependent cytotoxicity (ADCC), or an ability to block B7H3 in immunosuppression to enhance body immunity, a medication for promoting T lymphocyte, or a medication for improving production of cytokine such as IFN-γ in T lymphocyte.

The present disclosure further relates to a monoclonal antibody conjugate, including a monoclonal antibody and a conjugating part, wherein the monoclonal antibody is one of the previously mentioned anti-B7H3 monoclonal antibodies or their antigen-binding fragments, and the conjugating part is selected from one or more of a radionuclide, a medication, a toxin, a cytokine, a cytokine receptor fragment, an enzyme, fluorescein, and biotin.

The present disclosure further relates to use of the monoclonal antibody conjugate in preparation of the following medication: a medication for regulating B7H3 activity or B7H3 level, a medication having significant cellular internalization (allowing selective tumor killing or inhibition through toxin conjugation), a medication having significant antibody-dependent cytotoxicity (ADCC), a medication for blocking B7H3 in immunosuppression to enhance body immunity, a medication for promoting T lymphocyte, or a medication for improving production of cytokine such as IFN-γ in T lymphocyte.

The present disclosure further relates to use of the foregoing monoclonal antibody conjugate in preparation of a medication for preventing, treating, and/or adjuvantly treating a tumor.

The present disclosure further relates to an expression vector, including a nucleic acid molecule of the antibody.

The present disclosure further relates to an expression vector, including a nucleic acid molecule of the foregoing anti-B7H3 monoclonal antibody or the antigen-binding fragment thereof.

The term "vector" refers to a nucleic acid delivery vehicle into which a polynucleotide can be inserted. When a vector enables an inserted polynucleotide-encoded protein to be expressed, the vector is referred to as an expression vector. The vector can enter a host cell via transformation, transduction, or transfection, so that an element of a genetic material carried by the vector is expressed in the host cell. The vector is well-known to a person skilled in the art and includes, but is not limited to, a plasmid, a phagemid, a cosmid, an artificial chromosome (for example, a yeast artificial chromosome (YAC), a bacterial artificial chromosome (BAC), or a P1 derived artificial chromosome (PAC)), a phage (λ phage or M13 phage), and an animal virus. An animal virus that can serve as the vector includes, but is not limited to, retrovirus (including lentivirus), adenovirus, adeno-associated virus, herpesvirus (for example, herpes simplex virus), poxvirus, baculovirus, papillomavirus, and papillomavirus (for example, SV40). In some embodiments, the vector in the present disclosure includes a regulatory element commonly used in genetic engineering, for example, the enhancer, the promoter, an internal ribosome entry site (IRES) and another expression control element (for example, a transcription termination signal, or a polyadenylation signal and a polyU sequence).

In the present disclosure, the vector can be a composition, for example, a mixture of multiple plasmids, where different plasmids carry part of the antibody or the antigen-binding fragment.

The present disclosure further relates to a pharmaceutical composition, including the foregoing antibody or a (at least one) functional fragment thereof, and a pharmaceutical carrier.

As used herein, the "pharmaceutical carrier" includes any material that allows a component to remain biologically active when combined with the active component and that does not react with an immune system of the subject. An example includes, but is not limited to, any one of a standard drug carrier (for example, a phosphate buffered saline solution, water, an emulsion (for example, an oil/water emulsion)) and various types of wetting agents. A composition containing such carrier is prepared in a well-known conventional method (refer to, for example, Remington's Pharmaceutical Sciences, 18th edition, A. Gennaro, ed., Mack Publishing Co., Easton, PA, 1990; and Remington, The Science and Practice of Pharmacy, 21st edition, Mack Publishing, 2005).

The present disclosure further relates to use of the foregoing antibody or the functional fragment, nucleic acid molecule, expression vector, host cell, and pharmaceutical composition thereof in preparing B7H3 immunomodulatory medications.

The embodiments of the present disclosure are described in detail below with reference to examples:

### Example 1

### Immunization and Cell Fusion in Mice for Producing Anti-B7H3 Antibodies

Human B7H3 (NCBI Reference Sequence: NM_001024736.2) extracellular domain and mFc fusion protein (B7H3-ECD-mFc) were used as antigens, and fully emulsified with an equal volume of complete Freund's adjuvant (Sigma, Cat. No.: F5581), 6-8 week old Balb/c mice (Zhaoyan (Suzhou) New Drug Research Center Co., Ltd.) were subcutaneously immunized with a dose of 50 µg antigen per mouse. Subsequently, the mice were immunized subcutaneously three times with the same dose of antigen fully emulsified in incomplete Freund's adjuvant (Sigma, Cat. No.: F5506) every 2 weeks. After three immunizations, serum titers of the mice were measured, and the mice were booster immunized once via intraperitoneal injection 3 days before the fusion. Using PEG Hybri-Max (Sigma, Cat.: 7181) as a fusion agent, splenocytes of the mice and SP2/0 cells were mixed in a ratio of 4:1. Fused cells were added into a 96-well plate (1×10⁵ cells/well), and each well contained 0.1 mL of 1X HAT medium (Invitrogen, Cat. No.: 21060-017). 0.1 mL of HT (Invitrogen, Cat. No.: 11067-030) medium was added on day 3, the medium was pipetted out of the 96-well plate on day 7, and 0.2 mL of fresh HT medium was added. The supernatant was collected on day 9 for various screening and tests.

### Example 2

### Antigen Binding Ability of Hybridoma Antibodies and Subcloning

The binding ability of hybridoma antibodies was tested using ELISA and FACS, along with subcloning via the limiting dilution method::
(1) Screening of B7H3-binding positive clones through ELISA: 50 µL of B7H3-hFc (final concentration: 2 µg/mL) was used to coat each well of a 96-well ELISA plate (Corning, Cat. No.: 9018), and incubated overnight at room temperature. After the ELISA plate was rinsed with washing buffer (PBS+0.05% Tween20) 3 times, blocking buffer (PBS+2%BSA (Sigma, Cat. No.: V90093)) was added and the ELISA plate was incubated at room temperature for 1 hour. The ELISA plate was rinsed with washing buffer 3 times; and hybridoma supernatant was added, and the ELISA plate was incubated at room temperature for 1 hour and rinsed 3 times. 100 µL of 10,000-fold diluted HRP-conjugated goat anti-mouse IgG secondary antibody (Thermo, Cat. No.: 31432) was added to each well, and the ELISA plate was incubated at room temperature for 1 hour in the dark and rinsed 3 times. 100 µL of TMB (Biocytogen, Cat. No.: ES-002) was added to each well, and the ELISA plate was incubated at room temperature for color development for 2 minutes, 100 µL of reaction terminating solution (2N H2SO4) was added to each well to terminate the color reaction, and an OD450 value of each well was read using a microplate reader (Tecan Spark).
(2) Screening of B7H3-binding positive clones through FACS: 50 µL of the positive hybridoma supernatant or purified hybridoma antibody in the foregoing test was mixed with 50 µL of 293T-B7H3 cells (2×10⁵ cells/well), and a resulting mixture was added to a 96-well plate with a U-shaped bottom and left standing at 4°C for 1 hour, the 96-well plate was rinsed with FACS buffer (PBS+3% FCS) and centrifuged twice, then 400-fold diluted PE-labeled goat anti-mouse secondary antibody (Biolegend, Cat. No.: 405307) was added, the 96-well plate was incubated at 4°C for 30 minutes in the dark, rinsed with FACS buffer and centrifuged twice, and signal values of the cells in a PE channel were detected by using BD Accuri C6 flow cytometer.
(3) Subcloning: Positive multiclonal hybridoma cells identified by ELISA and FACS were subcloned using the limiting dilution method. The subclones were further detected and screened using ELISA and FACS to obtain positive hybridoma monoclonal clones.

### Antibody Purification, Concentration Determination, and Endotoxin Testing:

The positive monoclonal hybridoma cells were added to 50 mL of serum-free medium (Invitrogen, Cat. No.: 12045-076) and the serum-free medium was cultured for 8 to 9 days, and then centrifuged to collect supernatant. The monoclonal antibodies were purified through protein A affinity chromatography. The purified antibody samples were liquid-exchanged and concentrated in ultrafiltration centrifuge tubes (Millipore, Cat. No.: ACS500024). Protein concentration was determined in a BCA method and limulus reagent (Xiamen Bioendo Technology Co., Ltd.) was used to detect endotoxin content of purified antibody samples.

ELISA and FACS were conducted to detect the binding ability of purified antibody samples to B7H3. Results are shown in FIG. 1(a-b), FIG. 2(a-b) and Table 1. The selected hybridoma antibodies had higher affinity.

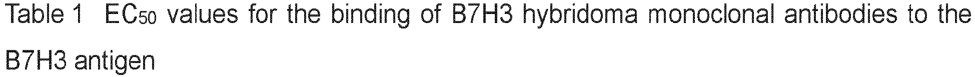

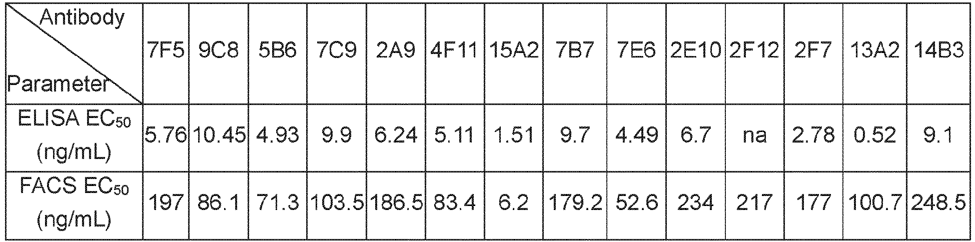

### Example 3

### Testing Intracellular Phagocytosis and B7H3 Blocking Action of Hybridoma Monoclonal Antibodies

Analysis of intracellular phagocytosis of antibodies: In a 96-well plate, 50 µL of 2×10⁶/mL PC9 cells and 50 µL of labeled B7H3 antibodies were added to each well, and the 96-well cell culture plate was incubated at 4°C for 1 hour. After the cell culture plate was rinsed with FACS buffer twice, Goat anti-mFc (Jackson, Cat. No.: 115-005-071) secondary antibody labeled with pH-dependent fluorescent dye CypHer5E (GE, Cat. No.: PA15401) was added, and the cell culture plate was incubated at 4°C for 0.5 hours and rinsed twice. A culture medium (1640+10% FBS) was added, the cell culture plate was put in an incubator and incubated for 3 hours, and then centrifuged to remove the culture medium. A residue was resuspended in PBS at pH 9.0, and CypHer5E signal of PC9 cells was detected through a BD C6 flow cytometer. Efficiency of entering the cells by B7H3 antibodies was calculated. As shown in FIG. 3(a-d) and FIG. 4(a-d), Jurkat-B7H3 and PC9 cells had significant endocytic activity for B7H3 antibodies, including 15A2, 5B6, 7C9, 2F7, 7B7, 2E10 and 4F11.

Testing of the effect of B7H3 hybridoma antibodies on secretion of cytokines by human PBMC cells: PBMC cells (TPCS, Cat. No.: PB025C) resuspended in complete medium (RPMI1640+10%FCS) was added into the 96-well plate (Corning, Cat. No.: 3799), then 40 ng/mL OKT3 (eBioscience, Cat. No.: 16-0037-85) was added, and the 96-well plate was incubated at 37°C for 72 hours. After counting, the activated PBMC cells were resuspended in complete culture medium (2.5×10⁵ cells/mL). In the 96-well plate, 100 µL of PBMC cells and 50 µL of B7H3 antibodies at different concentrations (initial concentration of 20 µg/mL, 10-fold serial dilution) were added to each well, the 96-well cell culture plate (Corning, Cat. No.: 3599) was placed in an incubator containing 5% CO₂ at 37°C for 48 hours, and the supernatant was collected. The concentrations of cytokines were detected using IFN-γ ELISA kit (R&D Systems, Cat. No.: DY285). As shown in FIG. 5, B7H3 antibodies including 5B6, 2F12, 2F7, 15A2, 13A2, 14B3, 4F11, 2A9, etc., can significantly promote the secretion of IFN-γ by PBMC cells.

### Example 4

### Cloning and Sequencing of B7H3 Antibody Variable Region Genes

B7H3 monoclonal hybridoma cell lines were lysed with TRIzon (Cwbiotech, Cat No: CW0580), and the total RNAs of hybridoma cells were extracted. RNA of hybridoma cells was reversely transcribed into cDNA by using HiFi Script cDNA Synthesis Kit (Cwbiotech, Cat No: CW2569). The cDNA was used as a template, degenerate primers were used to amplify heavy chain and light chain variable region genes of the antibody in a PCR method (Kettleborough et al. (1993) Eur. J Immunology 23: 206-211; Strebe et al. (2010) Antibody Engineering 1:3-14). After the product of the PCR amplification was ligated to T/A vector, DH5α competent cells were transformed, inoculated and cultured overnight at 37°C. Monoclonal was picked from the culture plate and cultured for amplification, plasmids were extracted, and the gene sequence of the antibody was identified. Based on the gene sequence of the antibody, complementarity-determining regions (CDRs) and framework regions of the antibody were analyzed. The B7H3 antibody sequence number is shown in Table 2, and specific sequence information is shown in the sequence listing.

**Table 2 B7H3 Antibody Sequence Number**

| **SEQ ID NO.** | **Description** | | **Amino acid sequence** |
|---|---|---|---|
| | Antibody | Sequence description | |
| 1 | 7F5 | Amino acid sequence of a heavy chain variable region | EVQLQQSGAELVRPGALVKLSCKTSGFNIKAYYI HWVKQRPEQGLEWIGWIDPENGNTFYDPKFQ GKASITADTSSNTAYLQLSSLTSEDAAVYYCTRS STRLRPFDYWGQGTTLTVSS |
| 2 | 7F5 | Amino acid sequence of a light chain variable region | DIVMTQAAFSNPVTLGTSASISCRSSKSLLHSN GITYLYWYLQKSGQSPQLLIYQMSNLASGVPDR FSSSGSGTDFTLRISRVEAEDVGVYYCAQNLEL YTFGGGTKLEIK |
| 3 | 9C8 | Amino acid sequence of a heavy chain variable region | EVQLQQSGPELVKPGASVKISCKTSGYTFTEYTI HWVKQSHGKSLEWIGGINPNSGGTTYNLKFKG KATLTVDKSSSTVYMELRSLTSEDSAVYYCARK EGFFTTDYWGQGTTLTVSS |
| 4 | 9C8 | Amino acid sequence of a light chain variable region | QTVLTQSPAIMSASPGEKVTMTCSASSSLSYIH WYRQKSGTSPKRWIYDTSNLATGVPVRFSGSG SGTSYSLTISSMEAEDAATYYCQQWHYNPPTF GGGTKLEIK |
| 5 | 5B6 | Amino acid sequence of a heavy chain variable region | QVQLQQSGAELARPGASVKMSCKTSGYTFITY TIHWVKQRPGQGLEWIGYINPTSDYANFNQKFK DKATLTADKSSRTAYMQLSSLTSEDSAVYYCAR WGTTVFQFYFDYWGQGTTLTVSS |
| 6 | 5B6 | Amino acid sequence of a light chain variable region | QIVLSQSPAILSASPGEKVTMTCRASQSLSYINW YQLKPGSSPKPWIYATSSLQSGVPARFSGSASG TSYSLTISRVEAEDAATYFCQHWSSDPPTIGGG TKLEIK |
| 7 | 7C9 | Amino acid sequence of a heavy chain variable region | EVQLQQSGTELVRPGALVKLSCKASALNIKDFY MHWVKQRPERGLEWIGWIDPDNGNTIYDPKFQ GKASITADISSSTAYLQLSSLTSEDTAVYYCARSS TRQRPFDYWGQGTTLTVSS |
| 8 | 7C9 | Amino acid sequence of a light chain variable region | DIVMTQSHKVMSTSVGDRVSITCKASQDVRTG VAWYQQKPGQSPKLLIYSAFYRYTGVPDRFTVS GSGADFTFTITSVQAEDLAVYYCQQYYSIPWTF GGGTKLEIK |
| 9 | 2A9 | Amino acid sequence of a heavy chain variable region | EVQLQQSGPELVKPGASVKISCKTSGYTFTEYI MHWVKQSRGKSLEWIGGINPNSGGTTYNEKFK GKATLTVDKSASTAYMELRSLTSEDSAIYYCARR MPYWYFDVWGAGTTVTVSS |
| 10 | 2A9 | Amino acid sequence of a light chain variable region | QIVLTQSPAIMSASPGEKVTMTCSASSSVSYIH WYRQKSGTSPKRWVYDTSKLASGVPARFSGS GSGTSYSLTISSMEAEDAATYYCQQWVSNPLTF GAGTKLELK |
| 11 | 4F11 | Amino acid sequence of a heavy chain variable region | EVQLQQSGPELVKPGASVKLSCKTSGYTFTEYI MHWVKQSHGKSLEWIGGINPNTGGITYNQKFK DKATLTVDKSSSTAYMELRSLTSDDSAVYFSAR AGGNFLWYFDVWGAGTTVTVSS |
| 12 | 4F11 | Amino acid sequence of a light chain variable region | QIVLTQSPATMSASPGEKVTMTCSASSSVSYMH WYQQKSDMSPKRWLYDTSKLASGVPARFSGS GSGTSYSLAISSMEAEDAATYYCQQWNSNPLT FGTGTKLELK |
| 13 | 15A2 | Amino acid sequence of a heavy chain variable region | QVQLQQSGPEVVKPGALVRISCKASGYTFTSY DINWVKQRPGQGLEWIGWIDPGDGSTKYNEKF KGKATLTADKSSSTAYMQFSSLTSENSAVYFCV RRGFDYWGQGTTLTVSS |
| 14 | 15A2 | Amino acid sequence of a light chain variable region | QILLTQSPAIMSASPGEKVTMTCRTSSSVSYLH WFQQKPGSSPKLWIHDTSNLASGFPARFSGSG SGTSYSLIISSMEAEDAATYYCHQRSSYPWTFG GGTKLEIK |
| 15 | 7B7 | Amino acid sequence of a heavy chain variable region | EVKLVESGGGLVRPGGSLKLSCAASGFTFSSYA VSWVRQTPEKRLEWVASISGGGIYIYYPDSVKG RFTISRDNAKNNLYLQMNSLTPDDTALYYCARH GGAGYFDYWGQGTTLTVSS |
| 16 | 7B7 | Amino acid sequence of a light chain variable region | DSLMTQSPASLSASVGETVTITCRGSESVHSYL AWYQQKQGKSPQLLVYNAKTLQSGVPSRFSGS GSGTQFSLRINSLQPEDFGTYYCQQHYGSPPW TFGGGTILEIK |
| 17 | 7E6 | Amino acid sequence of a heavy chain variable region | QVQLQQSGPELVRPGVSVKISCKGSGYTFTDY AMHWVKQSHAKSLEWIGVINSYYGNTHYNQKF KGKATMTVDKSSSTAYMELARLTSEDSAIYYCA |
| | | | RAGGYSYGSSYRPDRFAYWGQGTLVIVSA |
| 18 | 7E6 | Amino acid sequence of a light chain variable region | DIQMTQSPASQSASLGESVTITCLASQTIGTWLA WYQQKPGKSPQLLIYAATNLADGVPSRFSGSG SGTKFSFKISSLQAEDFVSYYCQQLYSTPYTFG GGTKLEIK |
| 19 | 2E10 | Amino acid sequence of a heavy chain variable region | EVQLQQSGPELVKPGASVKISCKTSGYTFTEYT MHWVKQSHGKNLEWIGGINTDNGGTTYSQKFK DKATLTVDRSSSTAYMVLRSLTSEDSAVYYCAR SFNNYGPAWFAYWGQGALVTVSA |
| 20 | 2E10 | Amino acid sequence of a light chain variable region | DIVMTQSHKFMSTSIGDRVSITCKASQDVRSAV AWYQQRPGQSPKLLIYWASTRHTGVPARFTGS GSGTDYTLTINNVQSEDLAGYFCQQYSSYFTFG GGTKLEKK |
| 21 | 2F12 | Amino acid sequence of a heavy chain variable region | EVQLQQSGPELVKSGASVNISCKTSGYTFTENT MHWVKLSHGKSLEWIGGINPNNGGTTYNQKFK GKATLTVDKSSSTAYMELRSLTSEDSAVYYCAR RGRWPPDWYFDVWGAGTTVTVSS |
| 22 | 2F12 | Amino acid sequence of a light chain variable region | QIVLTQSPAIMSASPGEKVTMTCSASSSLSYMH WYQQKSGTSPKRWIYDTSKLASGVPARFSGSG SGTSYSLTISSMEAEDAATYYCQQWSSNPLTFG AGTKLELK |
| 23 | 2F7 | Amino acid sequence of a heavy chain variable region | QVQLQQPGAELVKPGASVKLSCKASGYTFTSY YIYWLKQRPGQGLEWIGGIKPSNGDTNFNAKF MNKATLTVDKSSSTAYMQLSSLTSEDSAIYYCTR FFYGSPYDFDVWGAGTTVTVSS |
| 24 | 2F7 | Amino acid sequence of a light chain variable region | NIVMTQSPKSMSMAVGERVTLTCKASENVDTH VSWYQQKPEQSPTLLMYGTSNRFTGVPDRFT GSGSATDFTLTISSVQAEDLADYHCGQSYRYPY TFGGGTKLEIK |
| 25 | 13A2 | Amino acid sequence of a heavy chain variable region | EVQLQQSGPELVKPGASVKISCKTSGYTFTEYV MHWVKQRHGQSLEWIGGINPNTGGTTYNQKF EGKATLTVDKSSSSAYMEFRSLTSEDSAVYYCA RRMPFGISAMDYWGQGTSVTVSS |
| 26 | 13A2 | Amino acid sequence of a light chain variable region | QIVLTQSPAIMSASPGEKVTMTCSASSSVSYIH WFQQKSGTSPKRWISETSKLASGVPARFSGSG SGTSYSLTISSMGAEDAATYYCHQWISYPPTFG AGTKLELK |
| 27 | 14B3 | Amino acid sequence of a heavy chain variable region | QVQLQQSGPELVRPGVSVKISCKGSGYTFTDH AIHWVKQSHAKSLEWIGVINTYSGNTNFNQKLK GKATMTVDKSSSTAYMELARLTSEDSAIYYCAR GAHSSGYPYWYFDVWGAGTTVTVSS |
| 28 | 14B3 | Amino acid sequence of a light chain variable region | QIVLSQSPAILSASAGEKVTMTCRASSSVSYMH WYQQKPGSSPKPWIYATSNLASGVPARFSGSG SGTSYSLTISRVEAEDAATYYCQQWSTNPFTFG SGTKLEMK |
| 29 | 7F5 | Amino acid sequence of a heavy chain CDR1 region | AYYIH |
| 30 | 7F5 | Amino acid sequence of a heavy chain CDR2 region | WIDPENGNTFYDPKFQG |
| 31 | 7F5 | Amino acid sequence of a heavy chain CDR3 region | SSTRLRPFDY |
| 32 | 7F5 | Amino acid sequence of a light chain CDR1 region | RSSKSLLHSNGITYLY |
| 33 | 7F5 | Amino acid sequence of a light chain CDR2 region | QMSNLAS |
| 34 | 7F5 | Amino acid sequence of a light chain CDR3 region | AQNLELYT |
| 35 | 9C8 | Amino acid sequence of a heavy chain CDR1 region | EYTIH |
| 36 | 9C8 | Amino acid sequence of a heavy chain CDR2 region | GINPNSGGTTYNLKFKG |
| 37 | 9C8 | Amino acid sequence of a heavy chain CDR3 region | KEGFFTTDY |
| 38 | 9C8 | Amino acid sequence of a light chain CDR1 region | SASSSLSYIH |
| 39 | 9C8 | Amino acid sequence of a light chain CDR2 region | DTSNLAT |
| 40 | 9C8 | Amino acid sequence of a light chain CDR3 region | QQWHYNPPT |
| 41 | 5B6 | Amino acid sequence of a heavy chain CDR1 region | TYTIH |
| 42 | 5B6 | Amino acid sequence of a heavy chain CDR2 region | YINPTSDYANFNQKFKD |
| 43 | 5B6 | Amino acid sequence of a heavy chain CDR3 region | WGTTVFQFYFDY |
| 44 | 5B6 | Amino acid sequence of a light chain CDR1 region | RASQSLSYIN |
| 45 | 5B6 | Amino acid sequence of a light chain CDR2 region | ATSSLQS |
| 46 | 5B6 | Amino acid sequence of a light chain CDR3 region | QHWSSDPPT |
| 47 | 7C9 | Amino acid sequence of a heavy chain CDR1 region | DFYMH |
| 48 | 7C9 | Amino acid sequence of a heavy chain CDR2 region | WIDPDNGNTIYDPKFQG |
| 49 | 7C9 | Amino acid sequence of a heavy chain CDR3 region | SSTRQRPFDY |
| 50 | 7C9 | Amino acid sequence of a light chain CDR1 region | KASQDVRTGVA |
| 51 | 7C9 | Amino acid sequence of a light chain CDR2 region | SAFYRYT |
| 52 | 7C9 | Amino acid sequence of a light chain CDR3 region | QQYYSIPWT |
| 53 | 2A9 | Amino acid sequence of a heavy chain CDR1 region | EYIMH |
| 54 | 2A9 | Amino acid sequence of a heavy chain CDR2 region | GINPNSGGTTYNEKFKG |
| 55 | 2A9 | Amino acid sequence of a heavy chain CDR3 region | RMPYWYFDV |
| 56 | 2A9 | Amino acid sequence of a light chain CDR1 region | SASSSVSYIH |
| 57 | 2A9 | Amino acid sequence of a light chain CDR2 region | DTSKLAS |
| 58 | 2A9 | Amino acid sequence of a light chain CDR3 region | QQWVSNPLT |
| 59 | 4F11 | Amino acid sequence of a heavy chain CDR1 region | EYIMH |
| 60 | 4F11 | Amino acid sequence of a heavy chain CDR2 region | GINPNTGGITYNQKFKD |
| 61 | 4F11 | Amino acid sequence of a heavy chain CDR3 region | AGGNFLWYFDV |
| 62 | 4F11 | Amino acid sequence of a light chain CDR1 region | SASSSVSYMH |
| 63 | 4F11 | Amino acid sequence of a light chain CDR2 region | DTSKLAS |
| 64 | 4F11 | Amino acid sequence of a light chain CDR3 region | QQWNSNPLT |
| 65 | 15A2 | Amino acid sequence of a heavy chain CDR1 region | SYDIN |
| 66 | 15A2 | Amino acid sequence of a heavy chain CDR2 region | WIDPGDGSTKYNEKFKG |
| 67 | 15A2 | Amino acid sequence of a heavy chain CDR3 region | RGFDY |
| 68 | 15A2 | Amino acid sequence of a light chain CDR1 region | RTSSSVSYLH |
| 69 | 15A2 | Amino acid sequence of a light chain CDR2 region | DTSNLAS |
| 70 | 15A2 | Amino acid sequence of a light chain CDR3 region | HQRSSYPWT |
| 71 | 7B7 | Amino acid sequence of a heavy chain CDR1 region | SYAVS |
| 72 | 7B7 | Amino acid sequence of a heavy chain CDR2 region | SISGGGIYIYYPDSVKG |
| 73 | 7B7 | Amino acid sequence of a heavy chain CDR3 region | HGGAGYFDY |
| 74 | 7B7 | Amino acid sequence of a light chain CDR1 region | RGSESVHSYLA |
| 75 | 7B7 | Amino acid sequence of a light chain CDR2 region | NAKTLQS |
| 76 | 7B7 | Amino acid sequence of a light chain CDR3 region | QQHYGSPPWT |
| 77 | 7E6 | Amino acid sequence of a heavy chain CDR1 region | DYAMH |
| 78 | 7E6 | Amino acid sequence of a heavy chain CDR2 region | VINSYYGNTHYNQKFKG |
| 79 | 7E6 | Amino acid sequence of a heavy chain CDR3 region | AGGYSYGSSYRPDRFAY |
| 80 | 7E6 | Amino acid sequence of a light chain CDR1 region | LASQTIGTWLA |
| 81 | 7E6 | Amino acid sequence of a light chain CDR2 region | AATNLAD |
| 82 | 7E6 | Amino acid sequence of a light chain CDR3 region | QQLYSTPYT |
| 83 | 2E10 | Amino acid sequence of a heavy chain CDR1 region | EYTMH |
| 84 | 2E10 | Amino acid sequence of a heavy chain CDR2 region | GINTDNGGTTYSQKFKD |
| 85 | 2E10 | Amino acid sequence of a heavy chain CDR3 region | SFNNYGPAWFAY |
| 86 | 2E10 | Amino acid sequence of a light chain CDR1 region | KASQDVRSAVA |
| 87 | 2E10 | Amino acid sequence of a light chain CDR2 region | WASTRHT |
| 88 | 2E10 | Amino acid sequence of a light chain CDR3 region | QQYSSYFT |
| 89 | 2F12 | Amino acid sequence of a heavy chain CDR1 region | ENTMH |
| 90 | 2F12 | Amino acid sequence of a heavy chain CDR2 region | GINPNNGGTTYNQKFKG |
| 91 | 2F12 | Amino acid sequence of a heavy chain CDR3 region | RGRWPPDWYFDV |
| 92 | 2F12 | Amino acid sequence of a light chain CDR1 region | SASSSLSYMH |
| 93 | 2F12 | Amino acid sequence of a light chain CDR2 region | DTSKLAS |
| 94 | 2F12 | Amino acid sequence of a light chain CDR3 region | QQWSSNPLT |
| 95 | 2F7 | Amino acid sequence of a heavy chain CDR1 region | SYYIY |
| 96 | 2F7 | Amino acid sequence of a heavy chain CDR2 region | GIKPSNGDTNFNAKFMN |
| 97 | 2F7 | Amino acid sequence of a heavy chain CDR3 region | FFYGSPYDFDV |
| 98 | 2F7 | Amino acid sequence of a light chain CDR1 region | KASENVDTHVS |
| 99 | 2F7 | Amino acid sequence of a light chain CDR2 region | GTSNRFT |
| 100 | 2F7 | Amino acid sequence of a light chain CDR3 region | GQSYRYPYT |
| 101 | 13A2 | Amino acid sequence of a heavy chain CDR1 region | EYVMH |
| 102 | 13A2 | Amino acid sequence of a heavy chain CDR2 region | GINPNTGGTTYNQKFEG |
| 103 | 13A2 | Amino acid sequence of a heavy chain CDR3 region | RMPFGISAMDY |
| 104 | 13A2 | Amino acid sequence of a light chain CDR1 region | SASSSVSYIH |
| 105 | 13A2 | Amino acid sequence of a light chain CDR2 region | ETSKLAS |
| 106 | 13A2 | Amino acid sequence of a light chain CDR3 region | HQWISYPPT |
| 107 | 14B3 | Amino acid sequence of a heavy chain CDR1 region | DHAIH |
| 108 | 14B3 | Amino acid sequence of a heavy chain CDR2 region | VINTYSGNTNFNQKLKG |
| 109 | 14B3 | Amino acid sequence of a heavy chain CDR3 region | GAHSSGYPYWYFDV |
| 110 | 14B3 | Amino acid sequence of a light chain CDR1 region | RASSSVSYMH |
| 111 | 14B3 | Amino acid sequence of a light chain CDR2 region | ATSNLAS |
| 112 | 14B3 | Amino acid sequence of a light chain CDR3 region | QQWSTNPFT |

### Example 5

### ADCC Activity Testing of Hybridoma Monoclonal Antibodies

ADCC (antibody-dependent cell-mediated cytotoxicity) activity of the antibodies was tested using the reporter gene method: 293T-B7H3, PC9 or DLD1 tumor cells were used as target cells, and Jurkat-mCD16.2-NF-kB or Jurkat-hCD16-NF-kB cells were used as effector cells. The activation signal of transcription factor NF-kB mediated by mCD16.2 or hCD16 was used to screen ADCC-positive clones. 50 µL of hybridoma antibodies that were positive in the above ELISA and FACS tests were mixed with 25 µL of 293T-B7H3, PC9 or DLD1 tumor cells (7.5 × 10⁴ cells/well), a resulting mixture was added to a 96-well plate, and then 25 µL of Jurkat-mCD6. 2-NF-kB cells (2.5 × 104 cells/well) were added, mixed well, and incubated in an incubator for 4 hours at 37°C. 25 µL of preheated Bright-Glo solution (Promega, Cat. No.: E2620) was added to each well and left standing for 3 minutes at room temperature in the dark, and the luminescence (Luminescence) signal value of each sample was measured by using a microplate reader (Tecan Spark).

Among hybridoma antibodies, mouse IgG1 antibodies had no ADCC activity. After gene sequencing, the inventors confirmed that among the 14 hybridoma antibodies, 9 hybridoma antibodies were based on mouse IgG1, and 5 hybridoma antibodies were based on IgG2b or IgG2a (see Table 3). FIG. 6a shows ADCC reporter gene test results of hybridoma antibodies by using Jurkat-B7H3 as target cells. As expected, 5 hybridoma antibodies based on IgG2b or IgG2a had ADCC activity, while 9 hybridoma antibodies based on mouse IgG1 were inactive. Therefore, as shown in FIG. 6b, for 9 hybridoma antibodies based on mouse IgG1, the inventors conducted Fc replacement and turned these antibodies into chimeric antibodies based on human IgG1. FIG. 7 and FIG. 8 reflect the ADCC reporter gene test results by using PC9 or DLD1 tumor cells as targeted cells. The antibody was in the form of a chimeric antibody based on human IgG1. Based on the test results of ADCC reporter genes, antibodies with relatively strong ADCC activity included 9C8, 5B6, 7C9, F5, 7E6, 2A9, and 4F11.

**Table 3 Fc Types of B7H3 Hybridoma Antibodies**

| | | | | | | | | | | | | | | |
|---|---|---|---|---|---|---|---|---|---|---|---|---|---|---|
| Abs | 2A9 | 13A2 | 4F11 | 9C8 | 5B6 | 7E6 | 7F5 | 2F7 | 7C9 | 2E10 | 7B7 | 14B3 | 2F12 | 15A2 |
| Fc | IgG2b | IgG2b | IgG1 | IgG1 | IgG1 | IgG1 | IgG1 | IgG2b | IgG1 | IgG2a | IgG2b | IgG1 | IgG1 | IgG1 |

### Example 6

### Humanizing B7H3 Antibodies 2A9, 4F11, 9C8, 15A2, 5B6 and 7B7

Considering the various characteristics of hybridoma antibodies, six B7H3 antibodies, namely 2A9, 4F11, 9C8, 15A2, 5B6, and 7B7, were selected for humanization.

Humanization of these B7H3 antibodies was achieved through the complementary-determining region grafting method. Firstly, IMGT database was queried for human germline antibody (germline antibody) sequences with the maximum sequence homology to the light chain and heavy chain variable regions of murine-derived antibodies 2A9, 4F11, 9C8, 15A2, 5B6 and 7B7. Germline IGKV3-11*01 was selected for humanization of the light chain variable region of the antibody 2A9, and IGHV1-2*02 was selected for humanization of the heavy chain variable region. Germline IGKV3-11*01 was selected for humanization of the light chain variable region of the antibody 4F11, and IGHV1-2*02 was selected for humanization of the heavy chain variable region. Germline IGKV3-11*01 was selected for humanization of the light chain variable region of the antibody 9C8, and IGHV1-46*01 was selected for humanization of the heavy chain variable region. Germline IGKV3-11*01 was selected for humanization of the light chain variable region of the antibody 15A2, and IGHV1-8*01 was selected for humanization of the heavy chain variable region. Germline IGKV1-39*01 was selected for humanization of the light chain variable region of the antibody 5B6, and IGHV1-69*02 was selected for humanization of the heavy chain variable region. Germline IGKV1-39*01 was selected for humanization of the light chain variable region of the antibody 7B7, and IGHV3-21*01 was selected for humanization of the heavy chain variable region. The CDR region of the murine-derived antibodies was retained, and the framework (framework) sequence of the murine-derived antibodies was replaced with a framework sequence of the human germline antibodies. A structural model of the murine antibodies was created to compare amino acids at each site within the framework regions of both humanized and murine antibodies. If the spatial structure of the CDR region was not damaged or changed when a human amino acid sequence was used at a specific site in the framework, the human amino acid sequence was used at the specific site, otherwise, the corresponding murine sequence was used at the site (i.e., a reverse mutation to the murine sequence).

Based on structural simulations, Ala at the 24^{th} position of a humanized heavy chain of the 2A9 antibody was reversely mutated to Thr, Met at the 48^{th} position was reversely mutated to Ile, Val at the 67^{th} position was reversely mutated to Ala, Met at the 69^{th} position was reversely mutated to Leu, Arg at the 71^{rd} position was reversely mutated to Val, and Thr at the 73^{rd} position was reversely mutated to Lys. Leu at 46^{th} position of a humanized light chain of 2A9 antibody was reversely mutated to Arg, Leu at the 47^{th} position was reversely mutated to Trp, Ile at the 48^{th} position was reversely mutated to Val, and Phe at the 71^{st} position was reversely mutated to Tyr. Ala at the 24^{th} position of a humanized heavy chain of the antibody 4F11 was reversely mutated to Thr, Met at the 48^{th} position was reversely mutated to Ile, Val at the 67^{th} position was reversely mutated to Ala, Met at the 69^{th} position was reversely mutated to Leu, Arg at the 71^{st} position was reversely mutated to Val, and Thr at the 73^{rd} position was reversely mutated to Lys. Leu at 46^{th} position of a humanized light chain of 4F11 antibody was reversely mutated to Arg, Leu at the 47^{th} position was reversely mutated to Trp, Ile at the 48^{th} position was reversely mutated to Val, and Phe at the 71^{st} position was reversely mutated to Tyr. Met at the 48^{th} position of a humanized heavy chain of the antibody 9C8 was reversely mutated to Ile, Val at the 67^{th} position was reversely mutated to Ala, Met at the 69^{th} position was reversely mutated to Leu, Arg at the 71^{st} position was reversely mutated to Val, and Thr at the 73^{rd} position was reversely mutated to Lys. Ile at 2^{nd} position of a humanized light chain of 9C8 antibody was reversely mutated to Thr, Leu at the 46^{th} position was reversely mutated to Arg, Leu at the 47^{th} position was reversely mutated to Trp, and Phe at the 71^{st} position was reversely mutated to Tyr. Met at the 48^{th} position of a humanized heavy chain of the antibody 15A2 was reversely mutated to Ile, Val at the 67^{th} position was reversely mutated to Ala, Met at the 69^{th} position was reversely mutated to Leu, Arg at the 71^{st} position was reversely mutated to Ala, and Thr at the 73^{rd} position was reversely mutated to Lys. Tyr at 36^{th} position of a humanized light chain of 15A2 antibody was reversely mutated to Phe, Leu at the 47^{th} position was reversely mutated to Trp, Tyr at the 49^{th} position was reversely mutated to His, Ile at the 58^{th} position was reversely mutated to Phe, and Phe at the 71^{st} position was reversely mutated to Tyr. Gly at the 27^{th} position of a humanized heavy chain of 5B6 antibody was reversely mutated to Tyr, Ser at the 30^{th} position was reversely mutated to Ile, Met at the 48^{th} position was reversely mutated to Ile, Val at the 67^{th} position was reversely mutated to Ala, and Ile at the 69^{rd} position was reversely mutated to Leu. Leu at 46^{th} position of a humanized light chain of 5B6 antibody was reversely mutated to Arg, Leu at the 47^{th} position was reversely mutated to Pro, Gly at the 66^{th} position was reversely mutated to Ala, Phe at the 71^{st} position was reversely mutated to Tyr, and Phe at the 98^{th} position was reversely mutated to Ile. Ser at the 49^{th} position of a humanized heavy chain of 7B7 antibody was reversely mutated to Ala. Ile at 2^{nd} position of a humanized light chain of 7B7 antibody was reversely mutated to Ser, and Ile at the 48^{th} position was reversely mutated to Val.

The amino acid sequence numbers of the heavy chain and light chain variable regions of the humanized antibody are SEQ ID NO: 113 and SEQ ID NO: 114, respectively. The amino acid sequence numbers of the heavy chain and light chain variable regions of the humanized antibody 4F11 are SEQ ID NO: 115 and SEQ ID NO: 116, respectively. The amino acid sequence numbers of the heavy chain and light chain variable regions of the humanized antibody 9C8 are SEQ ID NO: 117 and SEQ ID NO: 118, respectively. The amino acid sequence numbers of the heavy chain and light chain variable regions of the humanized antibody 15A2 are SEQ ID NO: 119 and SEQ ID NO: 120, respectively. The amino acid sequence numbers of the heavy chain and light chain variable regions of the humanized antibody 5B6 are SEQ ID NO: 121 and SEQ ID NO: 122, respectively. The amino acid sequence numbers of the heavy chain and light chain variable regions of the humanized antibody 7B7 are SEQ ID NO: 123 and SEQ ID NO: 124, respectively. The above humanized antibodies were constructed as the IgG1 subtype. The amino acid sequence information of the variable regions of the heavy and light chains of humanized antibodies is shown in Table 4.

Nucleic acid sequences encoding the light and heavy chains of humanized antibodies 2A9, 4F11, 9C8, 15A2, 5B6 and 7B7 were synthesized and inserted into expression vector pcDNA3.1. 200 mL of 293 cells (cell density: 1×10⁶/mL) were co-transfected with 0.1 mg of antibody light chain expression plasmids and 0.1 mg of antibody heavy chain expression plasmids, and cultured while shaken in a shake flask at 37°C for 6 days, and the supernatant was collected by centrifugation. Protein A was used to purify humanized antibodies, and the purified humanized antibodies were tested for activity.

**Table 4 Sequence Listing of Humanized B7H3 Antibodies**

| SEQ ID NO. | Description | | |
|---|---|---|---|
| | Humanized Antibodies | Sequence description | Amino acid sequence |
| 113 | hu2A9 | Humanized heavy chain | QVQLVQSGAEVKKPGASVKVSCKTSGYT FTEYIMHWVRQAPGQGLEWIGGINPNSG GTTYNEKFKGRATLTVDKSISTAYMELSRL RSDDTAVYYCARRMPYWYFDVWGQGTM VTVSS |
| 114 | hu2A9 | Humanized light chain | EIVLTQSPATLSLSPGERATLSCSASSSVS YIHWYQQKPGQAPRRWVYDTSKLASGIP ARFSGSGSGTDYTLTISSLEPEDFAVYYC QQWVSNPLTFGQGTKLEIK |
| 115 | hu4F11 | Humanized heavy chain | QVQLVQSGAEVKKPGASVKVSCKTSGYT FTEYIMHWVRQAPGQGLEWIGGINPNTG GITYNQKFKDRATLTVDKSISTAYMELSRL RSDDTAVYYCARAGGNFLWYFDVWGQG TMVTVSS |
| 116 | hu4F11 | Humanized light chain | EIVLTQSPATLSLSPGERATLSCSASSSVS YMHWYQQKPGQAPRRWLYDTSKLASGI PARFSGSGSGTDYTLTISSLEPEDFAVYY CQQWNSNPLTFGQGTKLEIK |
| 117 | hu9C8 | Humanized heavy chain | QVQLVQSGAEVKKPGASVKVSCKASGYT FTEYTIHWVRQAPGQGLEWIGGINPNSG GTTYNLKFKGRATLTVDKSTSTVYMELSS LRSEDTAVYYCARKEGFFTTDYWGQGTL VTVSS |
| 118 | hu9C8 | Humanized light chain | ETVLTQSPATLSLSPGERATLSCSASSSLS YIHWYQQKPGQAPRRWIYDTSNLATGIPA RFSGSGSGTDYTLTISSLEPEDFAVYYCQ QWHYNPPTFGGGTKVEIK |
| 119 | hu15A2 | Humanized heavy chain | QVQLVQSGAEVKKPGASVKVSCKASGYT |
| | | | FTSYDINWVRQATGQGLEWIGWIDPGDG STKYNEKFKGRATLTANKSISTAYMELSSL RSEDTAVYYCVRRGFDYWGQGTLVTVSS |
| 120 | hu15A2 | Humanized light chain | EIVLTQSPATLSLSPGERATLSCRTSSSVS YLHWFQQKPGQAPRLWIHDTSNLASGFP ARFSGSGSGTDYTLTISSLEPEDFAVYYC HQRSSYPWTFGGGTKVEIK |
| 121 | hu5B6 | Humanized heavy chain | QVQLVQSGAEVKKPGSSVKVSCKASGYT FITYTIHWVRQAPGQGLEWIGYINPTSDY ANFNQKFKDRATLTADKSTSTAYMELSSL RSEDTAVYYCARWGTTVFQFYFDYWGQ GTLVTVSS |
| 122 | hu5B6 | Humanized light chain | EIVLTQSPATLSLSPGERATLSCRASQSLS YINWYQQKPGKAPKPWIYATSSLQSGVP SRFSGSASGTDYTLTISSLQPEDFATYYC QHWSSDPPTIGGGTKVEIK |
| 123 | hu7B7 | Humanized heavy chain | EVQLVESGGGLVKPGGSLRLSCAASGFT FSSYAVSWVRQAPGKGLEWVASISGGGI YIYYPDSVKGRFTISRDNAKNSLYLQMNS LRAEDTAVYYCARHGGAGYFDYWGQGT LVTVSS |
| 124 | hu7B7 | Humanized light chain | DSQMTQSPSSLSASVGDRVTITCRGSES VHSYLAWYQQKPGKAPKLLVYNAKTLQS GVPSRFSGSGSGTDFTLTISSLQPEDFAT YYCQQHYGSPPWTFGGGTKVEIK |

### Example 7

### Binding of Humanized B7H3 Antibodies to B7H3

The binding activity of humanized B7H3 antibody samples to B7H3 protein was assessed using ELISA and FACS, referring to the method detailed in Example 2. The results of humanized antibodies hu2A9, hu4F11, hu5B6, hu9C8, hu15A2 and hu7B7 are shown in Table 5 and Table 6 and FIG. 9 and FIG. 10. Overall, the humanized antibodies according to the present disclosure retained their high antigen affinity from before humanization, particularly notable in hu15A2, hu7B7, and hu5B6.

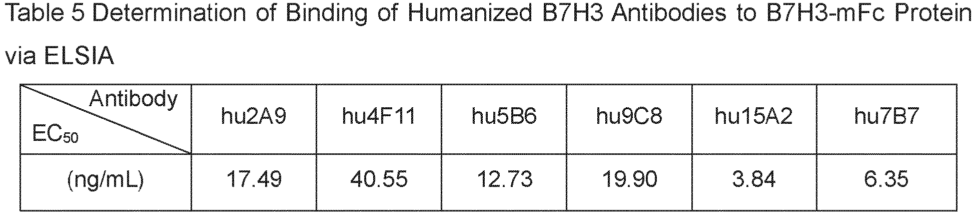

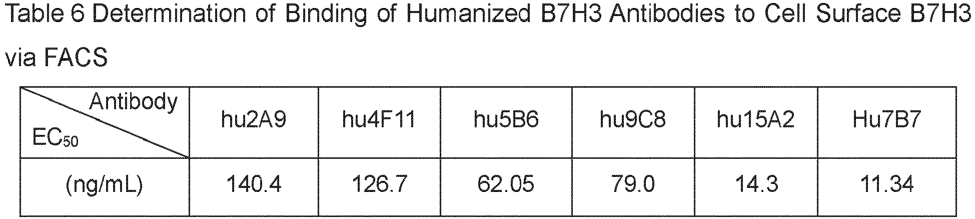

### Example 8

### Detection of Cellular Internalization of Humanized B7H3 Antibodies

For a specific measurement method, see Example 3. The results are shown in Table 7 and FIG. 11, showing that the humanized B7H3 antibodies had significant cellular internalization by PC9 cells. Compared with the positive control groups (MGA018 from MacroGenics and 8H9 from MSKCC), the cellular internalization of the antibody in the present disclosure was much stronger than that of 8H9. In addition, hu15A2 and hu7B7 also outperformed MGC018.

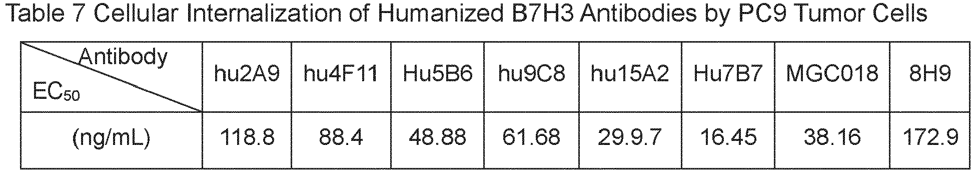

### Example 9

### Cytotoxic Effect of Toxin-Conjugated Humanized B7H3 Antibodies (B7H3-ADC) on Calu-6 Tumor Cells

Vc-MMAE was conjugated to the B7H3 antibodies in a chemical conjugation method. TCEP was used to reduce B7H3 antibodies in a molar ratio of 3:1, and a resulting mixture was incubated at 37°C for 45 minutes and subjected to ultrafiltration to remove TCE. The antibodies were resuspended in 0.5 mL of PBS, Vc-MMAE was added to the B7H3 antibodies in a mole ratio of 6 to 1, a resulting mixture was incubated at 4°C for 120 minutes and subjected to ultrafiltration to remove unconjugated Vc-MMAE, and the antibody concentration was measured for later use. 100 µL of PC9 (5000 cells per well) and 100 µL serially diluted B7H3-ADC were added to a 96-well plate. After incubation at 37°C for 5 days, × µL of CellTiter-Glo was added to each well, fluorescence intensity was measured, and an inhibition ability of the antibody against tumor cell proliferation was calculated. The results are shown in FIG. 12 and Table 8. Humanized B7H3 antibodies conjugated with MMAE toxin had a significant toxic effect on Calu-6 tumor cells, and was more active than the positive control group (MGC018-MMAE) from MacroGenics.

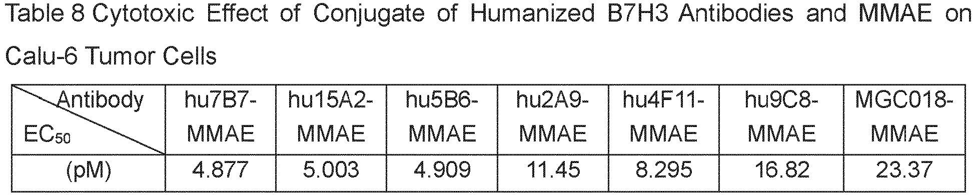

### Example 10

### Detection of ADCC Effect of Humanized B7H3 Antibody

For a specific measurement method, see Example 5. The results are shown in Table 9 and FIG. 13, showing that the humanized B7H3 antibodies had significant ADCC activity. MGA271 (MG-Ab) is an antibody from Macrogenics. Compared with MGA271 (MG-Ab), humanized 9C8, 5B6, 2A9 and 4F11 antibodies had lower EC₅₀ values, indicating better ADCC activity.

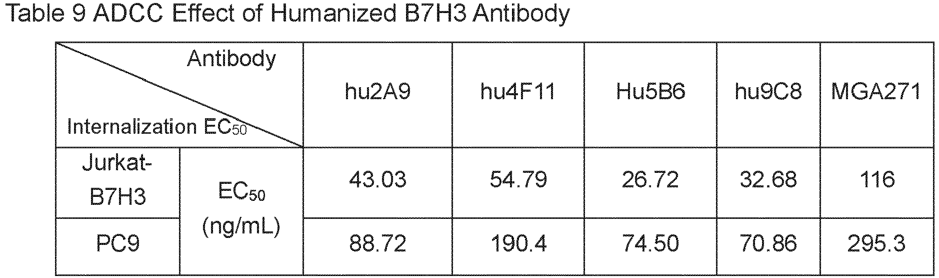

### Example 11

### ADCC Cytotoxic Effect of NK92MI-hCD16 Cells on Jurkat-B7H3 and PC9 Cells Mediated by Humanized B7H3 Antibodies

NK92MI-hCD16 was used to simulate NK cells to evaluate tumor-killing activity of B7H3 antibodies. 25 µL of PC9 (0.5×10⁵ cells per well) stained with CFSE and 25 µL of NK92MI-hCD16 (1×10⁵ cells per well) were mixed, and 50 µL of serially diluted B7H3 antibodies was added. After the 96-well plate was incubated for 4 hours at 37°C, 5 µL of 7-AAD was added to each well and the 96-well plate was incubated at room temperature for 10 minutes. The 96-well plate was centrifuged to remove the supernatant and resuspended. A flow cytometer was used to detect a ratio of cells positive for both 7-AAD and CFSE to CFSE-positive cells to obtain the B7H3 antibody-mediated NK cell killing activity. The results are shown in FIG. 14. Humanized B7H3 antibodies 9C8, 5B6, 2A9 and 4F11 could significantly cause an ADCC killing effect of NK92MI-hCD16 cells on PC9 tumor cells.

### Example 12

### Promoting Effect of Humanized B7H3 Antibodies on Secretion of Cytokines by Human PBMC Cells

For a specific measurement method, see Example 3. The results are shown in FIG. 15. Humanized B7H3 antibodies 5B6, 2A9 and 4F11 had a significant promoting effect on secretion of IFN-γ by human PBMC cells.

The foregoing embodiments are only preferred embodiments of the present disclosure and cannot be construed as a limitation on the protection scope of the present disclosure. That is, any simple equivalent changes and modifications made in accordance with the claims of the present disclosure and content of the utility model still fall within the protection scope of the present disclosure.

## Claims

1. An anti-B7H3 monoclonal antibody or an antigen-binding fragment thereof, comprising a heavy chain CDR and a light chain CDR, wherein the heavy chain CDR and the light chain CDR are selected from one or more groups of CDR combinations denoted as a to n:
| Combination name | Heavy chain CDR1 | Heavy chain CDR2 | Heavy chain CDR3 | Light chain CDR1 | Light chain CDR2 | Light chain CDR3 |
|---|---|---|---|---|---|---|
| a | SEQ ID NO: 29 | SEQ ID NO: 30 | SEQ ID NO: 31 | SEQ ID NO: 32 | SEQ ID NO: 33 | SEQ ID NO: 34 |
| b | SEQ ID NO: 35 | SEQ ID NO: 36 | SEQ ID NO: 37 | SEQ ID NO: 38 | SEQ ID NO: 39 | SEQ ID NO: 40 |
| c | SEQ ID NO: 41 | SEQ ID NO: 42 | SEQ ID NO: 43 | SEQ ID NO: 44 | SEQ ID NO: 45 | SEQ ID NO: 46 |
| d | SEQ ID NO: 47 | SEQ ID NO: 48 | SEQ ID NO: 49 | SEQ ID NO: 50 | SEQ ID NO: 51 | SEQ ID NO: 52 |
| e | SEQ ID NO: 53 | SEQ ID NO: 54 | SEQ ID NO: 55 | SEQ ID NO: 56 | SEQ ID NO: 57 | SEQ ID NO: 58 |
| f | SEQ ID NO: 59 | SEQ ID NO: 60 | SEQ ID NO: 61 | SEQ ID NO: 62 | SEQ ID NO: 63 | SEQ ID NO: 64 |
| 9 | SEQ ID NO: 65 | SEQ ID NO: 66 | SEQ ID NO: 67 | SEQ ID NO: 68 | SEQ ID NO: 69 | SEQ ID NO: 70 |
| h | SEQ ID NO: 71 | SEQ ID NO: 72 | SEQ ID NO: 73 | SEQ ID NO: 74 | SEQ ID NO: 75 | SEQ ID NO: 76 |
| i | SEQ ID NO: 77 | SEQ ID NO: 78 | SEQ ID NO: 79 | SEQ ID NO: 80 | SEQ ID NO: 81 | SEQ ID NO: 82 |
| g | SEQ ID NO: 83 | SEQ ID NO: 84 | SEQ ID NO: 85 | SEQ ID NO: 86 | SEQ ID NO: 87 | SEQ ID NO: 88 |
| k | SEQ ID NO: 89 | SEQ ID NO: 90 | SEQ ID NO: 91 | SEQ ID NO: 92 | SEQ ID NO: 93 | SEQ ID NO: 94 |
| l | SEQ ID NO: 95 | SEQ ID NO: 96 | SEQ ID NO: 97 | SEQ ID NO: 98 | SEQ ID NO: 99 | SEQ ID NO: 100 |
| m | SEQ ID NO: 101 | SEQ ID NO: 102 | SEQ ID NO: 103 | SEQ ID NO: 104 | SEQ ID NO: 105 | SEQ ID NO: 106 |
| n | SEQ ID NO: 107 | SEQ ID NO: 108 | SEQ ID NO: 109 | SEQ ID NO: 110 | SEQ ID NO: 111 | SEQ ID NO: 112 |

2. The anti-B7H3 monoclonal antibody or the antigen-binding fragment thereof according to claim 1, comprising a heavy chain variable region and a light chain variable region, wherein the heavy chain variable region and the light chain variable region are selected from one or more groups of variable region combinations denoted as A to N:
| Combination name | Heavy chain variable region | Light chain variable region |
|---|---|---|
| A | SEQ ID NO: 1 | SEQ ID NO: 2 |
| B | SEQ ID NO: 3 | SEQ ID NO: 4 |
| C | SEQ ID NO: 5 | SEQ ID NO: 6 |
| D | SEQ ID NO: 7 | SEQ ID NO: 8 |
| E | SEQ ID NO: 9 | SEQ ID NO: 10 |
| F | SEQ ID NO: 11 | SEQ ID NO: 12 |
| G | SEQ ID NO: 13 | SEQ ID NO: 14 |
| H | SEQ ID NO: 15 | SEQ ID NO: 16 |
| I | SEQ ID NO: 17 | SEQ ID NO: 18 |
| J | SEQ ID NO: 19 | SEQ ID NO: 20 |
| K | SEQ ID NO: 21 | SEQ ID NO: 22 |
| L | SEQ ID NO: 23 | SEQ ID NO: 24 |
| M | SEQ ID NO: 25 | SEQ ID NO: 26 |
| N | SEQ ID NO: 27 | SEQ ID NO: 28 |

3. The anti-B7H3 monoclonal antibody or the antigen-binding fragment thereof according to claim 2, wherein the heavy chain variable region and the light chain variable region are humanized.

4. The anti-B7H3 monoclonal antibody or the antigen-binding fragment thereof according to claim 3, wherein the humanized heavy chain variable region and light chain variable region are selected from one or more groups of variable region combinations denoted as 1 to 6:
| Combination name | Heavy chain variable region | Light chain variable region |
|---|---|---|
| 1 | SEQ ID NO: 113 | SEQ ID NO: 114 |
| 2 | SEQ ID NO: 115 | SEQ ID NO: 116 |
| 3 | SEQ ID NO: 117 | SEQ ID NO: 118 |
| 4 | SEQ ID NO: 119 | SEQ ID NO: 120 |
| 5 | SEQ ID NO: 121 | SEQ ID NO: 122 |
| 6 | SEQ ID NO: 123 | SEQ ID NO: 124 |

5. The anti-B7H3 monoclonal antibody or the antigen-binding fragment thereof according to claim 4, comprising a constant region of human IgG1.

6. Use of the anti-B7H3 monoclonal antibody or the antigen-binding fragment thereof according to any one of claims 1 to 5 in preparation of a medication which is: a medication for regulating B7H3 activity or B7H3 level, a medication having significant cellular internalization (allowing selective tumor killing or inhibition through toxin conjugation), significant antibody-dependent cytotoxicity (ADCC), or an ability to block an effect of B7H3 in immunosuppression to enhance body immunity, a medication for promoting T lymphocyte, or a medication for improving production of cytokine such as IFN-γ in T lymphocyte.

7. A monoclonal antibody conjugate, comprising a monoclonal antibody and a conjugating part, wherein the monoclonal antibody is the anti-B7H3 monoclonal antibody or the antigen-binding fragment thereof according to any one of claims 1 to 5, and the conjugating part is selected from one or more of a radionuclide, a medication, a toxin, a cytokine, a cytokine receptor fragment, an enzyme, fluorescein, and biotin.

8. Use of the monoclonal antibody conjugate according to claim 7 in preparation of a medication which is: a medication for regulating B7H3 activity or B7H3 level, a medication having significant cellular internalization (allowing selective tumor killing or inhibition through toxin conjugation), a medication having significant antibody-dependent cytotoxicity (ADCC), a medication for blocking an effect of B7H3 in immunosuppression to enhance body immunity, a medication for promoting T lymphocyte, or a medication for improving production of cytokine such as IFN-γ in T lymphocyte.

9. Use of the monoclonal antibody conjugate according to claim 7 in preparation of a medication for preventing, treating, and/or adjuvantly treating a tumor.

10. An expression vector, comprising a nucleic acid molecule of the anti-B7H3 monoclonal antibody or the antigen-binding fragment thereof according to any one of claims 1 to 5.

11. A pharmaceutical composition, comprising the anti-B7H3 monoclonal antibody or the antigen-binding fragment thereof according to any one of claims 1 to 5, and a pharmaceutical carrier.
